# EUROPEAN PATENT APPLICATION

(11) **EP 1 250 931 A1**
(43) Date of publication of application: **23.10.2002**
(21) Application number: 01109536.1
(22) Date of filing: 17.04.2001
(51) Int. Cl.: A61K 38/16, A61P 1/00, A61P 25/16, A61P 25/18, A61P 25/24, A61P 25/28

(54) **Novel use of neuronal calcium sensor-1 (NCS-1) in therapy of CNS disorders and in the development of therapeutic agents**

(71) Applicant: F. HOFFMANN-LA ROCHE AG, 4070 Basel (CH)
(72) Inventor: The designation of the inventor has not yet been filed
(74) Representative: VOSSIUS & PARTNER

(57) **Abstract**

Described are pharmaceutical compositions comprising compounds capable of modulating neuron-specific calcium sensor-1 (NCS-1) and their use for the treatment of CNS disorders such as Schizophrenia, Alzheimer's Disease, Parkinson's Disease, hyperactivity, and for improving cognition of the subject. Furthermore, the use of NCS-1 and corresponding cell based assays as research and working tools for a method of identifying and obtaining drug candidates for the therapy of the mentioned disorders or for improving cognition of a subject are provided.

## Description

The present invention relates to pharmaceutical compositions comprising compounds capable of modulating neuron-specific calcium sensor-1 (NCS-1) and their use for the treatment of CNS disorders such as Schizophrenia, Alzheimer's Disease, Parkinson's Disease, hyperactivity, and for improving cognition of the subject. Furthermore, the present invention relates to the use of NCS-1 and corresponding cell based assays as research and working tools for a method of identifying and obtaining drug candidates for the therapy of the mentioned disorders or for improving cognition of a subject. Novel drugs obtained by the method are also subject of the present invention.

Several documents are cited throughout the text of this specification by name. Full bibliographic citations may be found at the end of the specification immediately preceding the claims. Each of the documents cited herein (including any manufacturer's specifications, instructions, etc.) are hereby incorporated herein by reference; however, there is no admission that any document cited is indeed prior art as to the present invention.

The neuronal calcium sensor-1 (NCS-1) belongs to the intracellular neuronal calcium sensor family of EF-hand calcium-binding proteins (Braunewell and Gundelfinger, 1999), and is highly conserved throughout evolution, with orthologues identified in yeast, *Drosophila, C. elegans*, avian birds, rodents and humans (Nef, 1996). Its amino acid sequence is 100% conserved among vertebrates and shows only 25% divergence with *C. elegans*, or 28% with yeast (De Castro et al., 1995). NCS-1 is neuron-specific (Martone et al., 1999; Paterlini et al., 2000) and exhibits a high affinity for calcium of 300 nM (Cox et al., 1994), which is within the linear range of localized [Ca²⁺]ᵢ fluctuations in neurons (Fontana and Blaustein, 1993; Yazejian et al., 2000). The yeast, but not vertebrate (Bartlett et al., 2000), NCS-1 protein has been shown to interact with phosphatidylinositol 4-OH kinase (Hendricks et al., 1999). The vertebrate recombinant NCS-1 has been reported to activate G-protein receptor kinase 1 (De Castro et al., 1995; lacovelli et al., 1999), to regulate evoked exocytosis in neuroendocrine cells (McFerran et al., 1998), and to substitute for calmodulin (Schaad et al., 1996). Indeed, calmodulin-dependent targets such as 3':5'-cyclic nucleotide phosphodiesterase, calcineurin, nitric oxide synthase enzymes (*in vitro*), or the *Paramecium* calmodulin-dependent potassium channel (*in vivo*) are directly activated by NCS-1 (Schaad et al., 1996). Overexpression of frequenin, the *Drosophila* orthologue of NCS-1, causes a chronic facilitation of transmitter release at the neuromuscular junction of flies and frogs through unknown mechanisms (Olafsson et al., 1995; Pongs et al., 1993; Rivosecchi et al., 1994). However, the function of NCS-1, if any, in terms of particular phenotypic characteristics responsive to NCS-1 activity remained unknown.

The above-defined technical problem is solved by the present invention by providing the embodiments characterized in the claims. In particular, the present invention provides a novel pathway of CNS function via the neuronal calcium sensor NCS-1 which can be used as a novel target for therapeutic intervention.

Accordingly, in one aspect the present invention relates to a pharmaceutical composition comprising an agonist/activator or antagonist/inhibitor of neuron-specific calcium sensor-1 (NCS-1), and a pharmaceutically acceptable carrier.

In accordance with the present invention, the neuronal role of *ncs* genes *in vivo* has been characterized by loss-of-function genetics in a eukaryotic organism. To investigate the role of NCS-1 as a regulator of neuronal activity *in vivo, C. elegans* has been chosen as a model organism due to its simple nervous system and well-described neuronal circuitry with the ability to respond to diverse environmental stimuli such as touch, smell, taste or temperature.
The nucleotide and amino acid sequences of NCS-1 are described in the prior art, for example in the documents cited in background section above, the disclosure content of which is hereby incorporated the reference. Furthermore, the coding sequences of NCS-1 genes can be retrieved from public data bases, such as from NCBI; see for example accession numbers XM_005625 and NM_014286 describing NCS-1 as the Homo sapiens frequenin (Drosophila) homolog (FREQ), NM_019681 describing the Mus musculus NCS-1 or frequenin homolog, AL447416 describing a *Paramecium* homolog of NCS-1, AF020184 describing mouse neuronal calcium sensor-1 (NCS-1) mRNA, L27421 describing rat neuronal calcium sensor (NCS-1), L33680 describing Caenorhabditis elegans neuronal calcium binding protein (NCS-1), and L27420 describing and avian (Gallus gallus) neuronal calcium sensor (NCS-1) and references cited in the annotations. In order to avoid confusion, it should be noted that NCS-1 refers to the mammalian protein and frequenin to the drosophila protein but in reality NCS-1=frequenin, and frequenin=NCS-1.

As mentioned above, the present invention for the first time could establish a functional role of NCS-1 for the phenotype of a living organism using the model system *C. elegans.* On a radial temperature gradient *C. elegans* worms migrate, after conditioning with food, toward their cultivation temperature and move along this isotherm. This experience-dependent behavior is called isothermal tracking (IT). Experiments performed in accordance with the present invention surprisingly show that the neuron-specific calcium sensor-1 (NCS-1), a protein highly conserved through evolution, is essential for optimal IT behavior. *ncs-1* knockout animals show major defects in IT behavior, although their chemotactic, locomotor and thermal avoidance behaviors are normal. The knockout phenotype can be rescued by re-introducing wild-type NCS-1 into the AIY interneuron, a key component of the thermotaxis network. A loss of function form of NCS-1 incapable of binding calcium does not restore IT behavior, whereas NCS-1 overexpression enhances IT behavior performance levels, accelerates learning (faster acquisition), and produces a memory with slower extinction. Thus, proper calcium signaling via the neuronal calcium sensor NCS-1 defines a novel pathway essential for associative learning and memory. This finding also implicates that NCS-1 or compounds capable of modulating the activity of NCS-1 can be used for the treatment of disorders of the CNS, in particular those that display phenotypes related to altered behavior and loss of memory. Accordingly, the present invention provides the use of NCS-1 and compounds capable of modulating the activity or the amount of active NCS-1 for the ameliorate of CNS disorders which are related to the malfunction of the NCS-1 gene or its gene product. Furthermore, such compounds can be used for the treatment of symptoms of CNS disorders which are caused by mutant genes other than NCS-1 and/or are caused by the exposure to certain environmental conditions, for example stress, pollution, heat, poisoning, drug abuse, smoking, and the like. In addition, disorders resulting from aging processes such as loss of memory are expected to be effectively treated with compounds capable of modulating NCS-1 activity or by elevating the amount of active NCS-1 protein. The method of the present invention will help to identify and obtain such compounds which are drug candidates for the treatment of the mentioned disorders. Prominent examples of such disorders are Schizophrenia, Alzheimer's Disease, Parkinson's Disease, Major Depression, Bipolar Disorder, Anxiety Disorders, Appetite Disorders, Sleep Disorders, Insomnia, Attention Deficit Hyperactivity Disorder, drug abuse, and other.
Accordingly, the present invention relates to the use of an agonist/activator or of an antagonist/inhibitor of neuron-specific calcium sensor-1 (NCS-1) or a pharmaceutically acceptable salt thereof for the preparation of a composition for the treatment of a CNS disorder or for improving cognition of a subject. Preferably, said CNS disorder is Schizophrenia, Alzheimer's Disease, Parkinson's Disease or hyperactivity.

The terms "antagonist/inhibitor and agonist/activator" in accordance with the present invention include chemical agents that modulate the action of NCS-1, either through altering its enzymatic activity or through modulation of expression, e.g., by affecting transcription or translation. In some cases the antagonist/inhibitor or agonist/activator may also be a substrate or ligand binding molecule.
The term "activator," as used herein, includes both substances necessary for the NCS-1 to become active in the first place, and substances which merely accentuate its activity. The term "inhibitor" includes both substances which reduce the activity of the NCS-1 and these which nullify it altogether. When more than one possible activity is defined for NCS-1, for example calcium binding, increase of long-term potentiation in the hippocampus, facilitation of transmitter release and/or any other activity described in the background section above, the inhibitor or activator may modulate any or all of NCS-1 activities. An "antagonist" or "agonist" that modulates the activity of NCS-1 and causes for example a response in a cell based assay described below refers to a compound that alters directly or indirectly the activity NCS-1 or the amount of active NCS-1.

Typically, the effect of an antagonist is observed as a blocking of agonist-induced activation of calcium signalling. Antagonists include competitive as well as non-competitive antagonists. A competitive antagonist (or competitive blocker) interacts with or near the site specific for agonist binding. A non-competitive antagonist or blocker inactivates the function of the receptor by interacting with a site other than the agonist interaction site. Preferably, the antagonist/inhibitor and agonist/activator of NCS-1 are small chemical agents which directly interact with NCS-1. Therefore, there will preferably be a direct relationship between the molar amount of compound required to inhibit or stimulate NCS-1 activity and the molar amount of NCS-1 present or lacking in the cell.

Activators and inhibitors may be designed by structure-assisted computer modeling for example according to alpha-helix and alpha-helix forming regions ("alpha-regions"), beta-sheet and beta-sheet-forming regions ("beta-regions"), turn and turn-forming regions ("turn-regions"), coil and coil-forming regions ("coil-regions"), hydrophilic regions, hydrophobic regions, alpha amphipathic regions, beta amphipathic regions, flexible regions, surface-forming regions, substrate binding region, and high antigenic index regions. Such preferred regions include Garnier-Robson alpha-regions, beta-regions, turn-regions, and coil-regions, Chou-Fasman alpha-regions, beta-regions, and turn-regions, Kyte-Doolittle hydrophilic regions and hydrophobic regions, Eisenberg alpha and beta amphipathic regions, Karplus-Schulz flexible regions, Emini surface-forming regions, and Jameson-Wolf high antigenic index regions. Computer predictions can be made made using for example GCG-software derived from HGMP resource center Cambridge (Rice, 1995) Programme Manual for the EGCG package. (Cambridge, CB10 1RQ, England: Hinxton Hall).

In one embodiment of the pharmaceutical composition and the use of the present invention, the agonist/activator is or is derived from an NCS-1 polypeptide, an anti-NCS-1 antibody, a transcription regulator of the *ncs*-1 gene, a ligand binding molecule, a calcium mimetic or a derivative of a calmodulin activator.

Using known methods of protein engineering and recombinant DNA technology, variants may be generated to improve or alter the characteristics of the NCS-1 polypeptides. For instance, one or more amino acids can be deleted from the N-terminus or C-terminus of the protein without substantial loss of biological function. The authors of Ron, J. Biol. Chem. 268 (1993), 2984-2988, reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein. (Dobeli, J. Biotechnology 7 (1988), 199-216).
Moreover, ample evidence demonstrates that variants often retain a biological activity similar to that of the naturally occurring protein. For example, Gayle and coworkers (J. Biol. Chem. 268 (1993); 22105-22111) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]"; see Abstract. In fact, only 23 unique amino acid sequences, out of more than 3,500 nucleotide sequences examined, produced a protein that significantly differed in activity from wild-type. Furthermore, using the PESTFIND program (Rogers, Science 234 (1986), 364-368), PEST sequences (rich in proline, glutamic acid, serine, and threonine) can be identified, which are characteristically present in unstable proteins. Such sequences may be removed from the NCS-1 proteins in order to increase the stability and optionally the activity of the proteins. Methods for introducing such modifications in the nucleic acid molecules according to the invention are well-known to the person skilled in the art.
Thus, the present invention includes the use of NCS-1 polypeptide variants which show substantial biological activity. Such variants include deletions, insertions, inversions, repeats, and substitutions selected according to general rules known in the art so as have little effect on activity. For example, guidance concerning how to make phenotypically silent amino acid substitutions is provided in Bowie, Science 247 (1990), 1306-1310, wherein the authors indicate that there are two main strategies for studying the tolerance of an amino acid sequence to change.
Besides conservative amino acid substitution, variants of NCS-1 include (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability and/or solubility of the polypeptide (for example, polyethylene glycol), or (iv) fusion of the polypeptide with additional amino acids, such as an IgG Fc fusion region peptide, or leader or secretary sequence, or a sequence facilitating purification. Such variant polypeptides are deemed to be within the scope of those skilled in the art from the teachings herein.
For example, NCS-1 polypeptide variants containing amino acid substitutions of charged amino acids with other charged or neutral amino acids may produce proteins with improved characteristics, such as less aggregation. Aggregation of pharmaceutical formulations both reduces activity and increases clearance due to the aggregate's immunogenic activity; see, e.g. Pinckard, Clin. Exp. Immunol. 2 (1967), 331-340; Robbins, Diabetes 36 (1987), 838-845; Cleland, Crit. Rev. Therapeutic Drug Carrier Systems 10 (1993), 307-377.

An anti-NCS-1 antibody to be used in accordance with pharmaceutical compositions of the present invention can be monoclonal antibody, a polyclonal antibody, a single chain antibody, human or humanized antibody, primatized, chimerized or fragment thereof that specifically binds an NCS-1 peptide or polypeptide also including bispecific antibody, synthetic antibody, antibody fragment, such as Fab, Fv or scFv fragments etc., or a chemically modified derivative of any of these. The general methodology for producing antibodies is well-known and has been described in, for example, Köhler and Milstein, Nature 256 (1975), 494 and reviewed in J.G.R. Hurrel, ed., "Monoclonal Hybridoma Antibodies: Techniques and Applications", CRC Press Inc., Boco Raron, FL (1982), as well as that taught by L. T. Mimms et al., Virology 176 (1990), 604-619. Furthermore, antibodies or fragments thereof to the aforementioned peptides can be obtained by using methods which are described, e.g., in Harlow and Lane "Antibodies, A Laboratory Manual", CSH Press, Cold Spring Harbor, 1988.

Further sources for the basic structure of activators or inhibitors can be employed and comprise, for example, mimetic analogs of the NCS-1 polypeptide. Mimetic analogs of the NCS-1 polypeptide or biologically active fragments thereof can be generated by, for example, substituting the amino acids that are expected to be essential for the biological activity with, e.g., stereoisomers, i.e. D-amino acids; see e.g., Tsukida, J. Med. Chem. 40 (1997), 3534-3541. Furthermore, in case fragments are used for the design of biologically active analogs pro-mimetic components can be incorporated into a peptide to reestablish at least some of the conformational properties that may have been lost upon removal of part of the original polypeptide; see, e.g., Nachman, Regul. Pept. 57 (1995), 359-370. Furthermore, the NCS-1 polypeptide can be used to identify synthetic chemical peptide mimetics that bind to or can function as a ligand, substrate, binding partner or the receptor of the NCS-1 polypeptide as effectively as does the natural polypeptide; see, e.g., Engleman, J. Clin. Invest. 99 (1997), 2284-2292. For example, folding simulations and computer redesign of structural motifs of the protein of the invention can be performed using appropriate computer programs (Olszewski, Proteins 25 (1996), 286-299; Hoffman, Comput. Appl. Biosci. 11 (1995), 675-679). Computer modeling of protein folding can be used for the conformational and energetic analysis of detailed peptide and protein models (Monge, J. Mol. Biol. 247 (1995), 995-1012; Renouf, Adv. Exp. Med. Biol. 376 (1995), 37-45). In particular, the appropriate programs can be used for the identification of interactive sites of the NCS-1 polypeptide and its ligand or other interacting proteins by computer assistant searches for complementary peptide sequences (Fassina, Immunomethods 5 (1994), 114-120. Further appropriate computer systems for the design of protein and peptides are described in the prior art, for example in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, a three-dimensional and/or crystallographic structure of the NCS-1 protein can be used for the design of mimetic inhibitors of the biological activity of the protein of the invention (Rose, Biochemistry 35 (1996), 12933-12944; Rutenber, Bioorg. Med. Chem. 4 (1996), 1545-1558).
The structure-based design and synthesis of low-molecular-weight synthetic molecules that mimic the activity of the native biological polypeptide is further described in, e.g., Dowd, Nature Biotechnol. 16 (1998), 190-195; Kieber-Emmons, Current Opinion Biotechnol. 8 (1997), 435-441; Moore, Proc. West Pharmacol. Soc. 40 (1997), 115-119; Mathews, Proc. West Pharmacol. Soc. 40 (1997), 121-125; Mukhija, European J. Biochem. 254 (1998), 433-438.

It is also well known to the person skilled in the art, that it is possible to design, synthesize and evaluate mimetics of small organic compounds that, for example, can act as a substrate or ligand to the NCS-1 polypeptide. For example, it has been described that D-glucose mimetics of hapalosin exhibited similar efficiency as hapalosin in antagonizing multidrug resistance assistance-associated protein in cytotoxicity; see Dinh, J. Med. Chem. 41 (1998), 981-987.

The polynucleotides encoding NCS-1 can also serve as a target for activators and inhibitors. Activators may comprise, for example, proteins that bind to the mRNA of a gene encoding a NCS-1 polypeptide, thereby stabilizing the native conformation of the mRNA and facilitating transcription and/or translation, e.g., in like manner as Tat protein acts on HIV-RNA. Furthermore, methods are described in the literature for identifying nucleic acid molecules such as an RNA fragment that mimics the structure of a defined or undefined target RNA molecule to which a compound binds inside of a cell resulting in retardation of cell growth or cell death; see, e.g., WO 98/18947 and references cited therein. These nucleic acid molecules can be used for identifying unknown compounds of pharmaceutical and/or agricultural interest, and for identifying unknown RNA targets for use in treating a disease. Alternatively, for example, the conformational structure of the RNA fragment which mimics the binding site can be employed in rational drug design to modify known ligands to make them bind more avidly to the target. One such methodology is nuclear magnetic resonance (NMR), which is useful to identify drug and RNA conformational structures. Still other methods are, for example, the drug design methods as described in WO 95/35367, US-A-5,322,933, where the crystal structure of the RNA fragment can be deduced and computer programs are utilized to design novel binding compounds which can act as antibiotics.

Some genetic changes lead to altered protein conformational states. For example, some mutant NCS-1 proteins may possess a tertiary structure that renders them far less capable of facilitating calcium signaling. Restoring the normal or regulated conformation of mutated proteins is the most elegant and specific means to correct these molecular defects, although it may be difficult. Of particular interest in this regard are the following domains of NCS-1: The 3 functional calcium binding sites called EF-hands (EF) at amino acid positions 73-84 (EF2), 109-120 (EF3), 157-168 (EF4) and the surrounding amino acid sequences or positions interacting with the calcium binding site. In addition, the former calcium binding site (EF1), at position 36-47 might contribute to calcium binding or regulate NCS-1 function. Finally, the N-terminus (i.e. amino acids 1-8) may serve as a myristoylation site and could provide a regulatory function of NCS-1. Pharmacological manipulations thus may aim at restoration of wild-type conformation of the NCS-1 protein. Thus, the present invention also uses molecules which are capable of activating the wild-type, i.e. "NCS-1" or "anti-NCS-1" function of a NCS-1 protein.

Recombinant NCS-1 polynucleotides, antisense molecules, vectors incorporating such polynucleotides or antisense molecules can be produced by methods known to those skilled in molecular biology. For example, the choice of vectors which would depend on the function desired and include plasmids, cosmids, viruses, bacteriophages and other vectors used conventionally in genetic engineering. Methods which are well known to those skilled in the art can be used to construct various plasmids and vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1989), (1994). Alternatively, the polynucleotides and vectors can be reconstituted into liposomes for delivery to target cells. Relevant sequences can be transferred into expression vectors where expression of a particular polypeptide is required. Typical cloning vectors include pBscpt sk, pGEM, pUC9, pBR322 and pGBT9. Typical expression vectors include pTRE, pCAL-n-EK, pESP-1, pOP13CAT, pET, pGEX, pMALC, pPIC9, pBac.

In another embodiment of the pharmaceutical composition and the use of the present invention, the antagonist/inhibitor is or is derived from an NCS-1 polypeptide, an anti-NCS-1 antibody, an *ncs*-1 antisense nucleic acid molecule, a ligand binding molecule, a calcium chelator or a calmodulin inhibitor. Preferably, said antagonist/inhibitor interfere with calcium binding of NCS-1 or change the conformation/function of NCS-1.

The antibodies, nucleic acid molecules, inhibitors and activators used in the compositions of the present invention preferably have a specificity at least substantially identical to the binding specificity of the natural ligand or binding partner of the NCS-1 protein, in particular if NCS-1 stimulation is desired. An antibody or inhibitor can have a binding affinity to the NCS-1 protein of at least 10⁵ M⁻¹, preferably higher than 10⁷ M⁻¹ and advantageously up to 10¹⁰ M⁻¹ in case NCS-1 suppression should be mediated.
In a preferred embodiment, a suppressive antibody or inhibitor has an affinity of at least about 10⁻⁷ M, preferably at least about 10⁻⁹ M and most preferably at least about 10⁻¹¹ M; and a NCS-1 stimulating activator has an affinity of less than about 10⁻⁷ M, preferably less than about 10⁻⁶ M and most preferably in order of 10⁻⁵M.
In case of antisense nucleic acid molecules it is preferred that they have a binding affinity to those encoding the NCS-1 protein of at most 2-, 5- or 10-fold less than an exact complement of 20 consecutive nucleotides of the coding sequence.

Preferably, the agonist/activator and antagonist/inhibitior is not larger than the "bioavailability wall" of 500-600 Da in order to be able to cross the lipophilic cell membrane into the cell. On the other hand, in protein therapy it has been recently demonstrated that enzymes fused to part of a protein from the HIV virus can cross cell membranes while retaining their enzymatic activity in vivo in mice (Schwarze, Science 285 (1999), 1569-1572). It has been known for approximately ten years that the transactivating regulatory protein (TAT protein) from the HIV virus has an unusual ability to cross cell membranes without using receptors or transporters, or requiring ATP (Green and Loewenstein, Cell 55 (1988), 1179-1188). Although its exact mechanism is unknown, it has been shown that the protein transduction domain (PTD) of TAT opens a "hole" in the cell membrane lipid bilayer, pulling anything covalently attached through it, before closing it again. This is a specific process that does not otherwise damage the cell. Thus, a functional NCS-1 protein, anti-NCS-1 antibody or other compounds may be coupled to PTD via a linker in order to let them cross the cell membrane; see also for review DDT 4 (1999), 537.

In a further aspect, the present invention relates to a cell based method of identifying and obtaining a drug candidate for therapy of a CNS disorder or for improving cognition of a subject, said method comprising the steps of
(a) screening a cell, tissue or non-human animal with a compound to be screened under conditions to permit neuron-specific calcium sensor-1 (NCS-1) activity; and
(b) determining NCS-1 activity of said treated cell, tissue or non-human animal, wherein a difference in NCS-1 activity compared with a corresponding control cell, tissue or animal is indicative for a drug candidate; and optionally
(c) obtaining the drug candidate determined to alter NCS-1 activity in step (b).

The amount of time necessary for cellular contact with the compound is empirically determined, for example, by running a time course with a known NCS-1 modulator and measuring cellular changes as a function of time. The measurement means of the method of the present invention can be further defined by comparing a cell that has been exposed to a compound to an identical cell that has not been similarly expose to the compound. Alternatively two cells, one containing functional NCS-1 and a second cell identical to the first, but lacking functional NCS-1 could be both be contacted with the same compound and compared for differences between the two cells. This technique is also useful in establishing the background noise of these assays. One of average skill in the art will appreciate that these control mechanisms also allow easy selection of cellular changes that are responsive to modulation of functional NCS-1.

The term "cell" refers to at least one cell, but includes a plurality of cells appropriate for the sensitivity of the detection method. Cells suitable for the present invention may be bacterial, yeast, or preferably eukaryotic. The methods of this invention employ certain types of cells, certain observations of changes in aspects of the biological state of a cell, and certain comparisons of these observed changes. In the following, these cell types, observations, and comparisons are described in turn in detail.
The present invention makes use of three principal types of cells: wild-type cells, modified cells i.e. transgenic cells, compound- or drug-exposed cells. "Wild-type" cells are reference, or standard, cells used in a particular application or embodiment of the methods of this invention. Being only a reference cell, a wild-type cell, need not be a cell normally found in nature, and often will be a recombinant or genetically altered cell line. Usually the cells are cultured in vitro as a cell line or strain. Other cell types used in the particular application of the present invention are preferably derived from the wild-type cells. Less preferably, other cell types are derived from cells substantially isogeneic with wild-type cells. For example, wild-type cells might be a particular cell line of the yeast Saccharomyces cerevisiae, or a particular mammalian cell line (e.g., HeLa cells). Although, for simplicity this disclosure often makes reference to single cells (e.g., "screening a cell"), it will be understood by those of skill in the art that more often any particular step of the invention will be carried out using a plurality of genetically identical cells, e.g., from a cultured cell line. Two cells are said to be "substantially isogeneic" where their expressed genomes differ by a known amount that is preferably at less than 10% of genetic loci, more preferably at less that 1%, or even more preferably at less than 0.1%. Alternately, two cells can be considered substantially isogeneic when the portions of their genomes relevant to the effects of a drug of interest differ by the preceding amounts. It is further preferable that the differing loci be individually known. "Compound- or drug-exposed" cells are, briefly, either wild-type cells or modified cells that have been exposed to (a) compound(s) of interest, e.g., drug candidate(s).
"Modified cells" are derived from wild-type cells by modifications to a particular cellular constituent. Methods of modification are adaptable to this invention if they alter, either by increasing or decreasing, preferably only a single targeted cellular constituent, or less preferably at most only a few targeted cellular constituents (e.g., from 2 to 5 cellular constituents), that influence the aspect of the biological state of a cell measured in an embodiment of this invention. Preferable modification methods are capable of individually targeting and altering many measured cellular constituents relevant to an aspect of the biological state, and most preferably are capable of targeting and altering a substantial fraction of such cellular constituents. For example, preferable modification methods are capable of targeting and altering, e.g., a substantial fraction of all the genes, proteins, or protein activities in a cell, or at least a substantial fraction of those constituents relevant to characterizing the effects of a drug of interest. Normally, the modified will be a transgenic cell.
The above-described cells can also be comprised in a tissue or organism, i.e. non-human animal. General methods for the screening of compounds that have a desired effect on a cell or organism as measured in a specific assay are described in the prior art; see for example US-A-6,165,709 and references cited herein.
Cells, non-human animals and NCS-1 expression and/or knock out systems can be found in the prior art and adapted for the method of the present invention; see for example the documents cited in the background section.

The assay methods to determine compound modulation of functional NCS-1 can be in conventional laboratory format or adapted for high throughput. The term "high throughput" refers to an assay design that allows easy analysis of multiple samples simultaneously, and capacity for robotic manipulation. Another desired feature of high throughput assays is an assay design that is optimized to reduce reagent usage, or minimize the number of manipulations in order to achieve the analysis desired. Examples of assay formats include 96-well, 384-well or more-well plates, levitating dropplets, and "lab on a chip" microchannel chips used for liquid handling experiments. It is well known by those in the art that as miniaturization of plastic molds and liquid handling devices are advanced, or as improved assay devices are designed, that greater numbers of samples may be performed using the design of the present invention.
The cellular changes suitable for the method of the present invention comprise directly measuring changes in the function or quantity of NCS-1, or by measuring downstream effects of NCS-1 function, for example by measuring secondary messanger concentrations or changes in transcription or by changes in protein levels of genes that are transcriptionally influenced by NCS-1, or by measuring phenotypic changes in the cell. Preferred measurement means include changes in the quantity of NCS-1 protein, changes in the functional activiy of NCS-1, changes in the quantity of mRNA, changes in intracellular protein, changes in cell surface protein, or secreted protein, or changes in Ca²⁺, cAMP or GTP concentration. Changes in the quantity or functional activity of NCS-1 are described herein. Said functional activity is preferably calcium binding. Changes in the levels of mRNA are detected by reverse transcription polymerase chain reaction (RT-PCR), by differential gene expression or by microarrays. Immunoaffinity, ligand affinity, or enzymatic measurement quantitates changes in levels of protein in host cells. Protein-specific affinity beads or specific antibodies are used to isolate for example ³⁵S-methionine labelled or unlabelled protein. Labelled protein is analyzed by SDS-PAGE. Unlabelled protein is detected by Western blotting, cell surface detection by fluorescent cell sorting, cell image analysis, ELISA or RIA employing specific antibodies. Where the protein is an enzyme, the induction of protein is monitored by cleavage of a flourogenic or colorimetric substrate.

Where the endogenous gene encodes a soluble intracellular protein, changes in the endogenous gene may be measured by changes of the specific protein contained within the cell lysate. The soluble protein may be measured by the methods described herein.

The present invention is also directed to methods for screening for compounds that modulate the expression of DNA or RNA encoding NCS-1 as well as the function of NCS-1 protein *in vivo*. Compounds may modulate by increasing or attenuating the expression of DNA or RNA encoding NCS-1, or the function of NCS-1 protein. Compounds that modulate the expression of DNA or RNA encoding NCS-1 or the function of NCS-1 protein may be detected by a variety of assays. The assay may be a simple "yes/no" assay to determine whether there is a change in expression or function. The assay may be made quantitative by comparing the expression or function of a test sample with the levels of expression or function in a standard sample. Modulators identified in this process are useful as therapeutic agents.

The above-described methods can, of course, be combined with one or more steps of any of the above-described screening methods or other screening methods well known in the art. Methods for clinical compound discovery comprises for example ultrahigh-throughput screening (Sundberg, Curr. Opin. Biotechnol. 11 (2000), 47-53) for lead identification, and structure-based drug design (Verlinde and Hol, Structure 2 (1994), 577-587) and combinatorial chemistry (Salemme et al., Structure 15 (1997), 319-324) for lead optimization.
Once a drug has been selected, the method can have the additional step of repeating the method used to perform rational drug design using the modified drug and to assess whether said modified drug displays better affinity according to for example interaction/energy analysis.

In a preferred embodiment of the method of the present invention, said cell, tissue or non-human animal is a transgenic cell, tissue or non-human animal which displays a substantially reduced or enhanced level of neuron-specific calcium sensor-1 (NCS-1) activity compared to a corresponding wild-type animal.

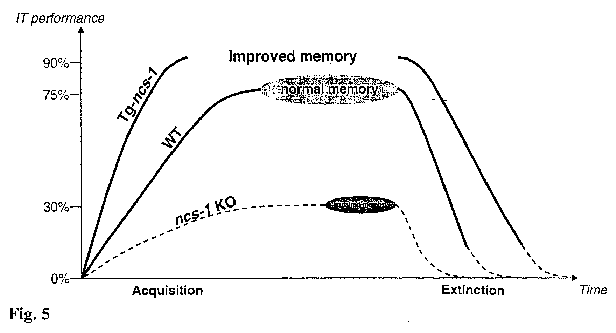

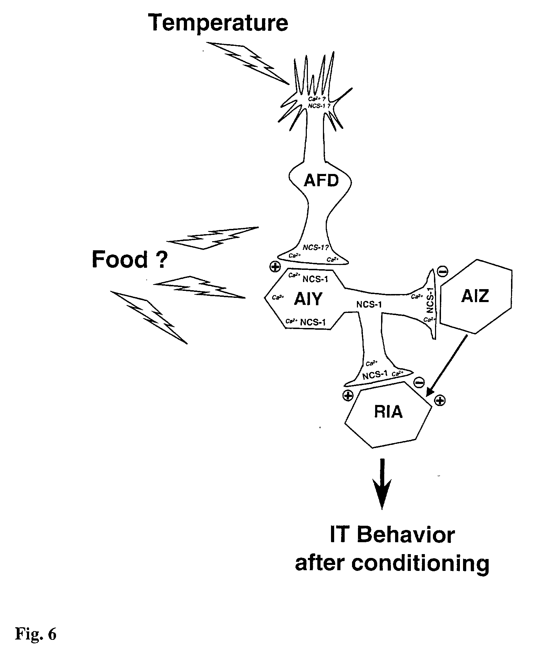

Preferably said substantially reduced or enhanced level of NCS-1 activity results in an altered and a typic response of the transgenic cell, tissue or non-human animal. An agonist/activator or antagonist/inhibitor will then be identified by observing whether a candidate compound is able at a certain concentration to revert the phenotypic response of said transgenic cell, tissue or non-human animal back to normal. In a particular preferred embodiment, said transgenic non-human animal displays a difference in behavior compared to a wild type non-human animal. In accordance with the present invention, it could be surprisingly shown that in *C. elegans* NCS-1 activity is linked with isothermal tracking (IT) behavior and also learning mechanisms. Reinforcement via faster acquisition together with higher final performance, not surprisingly, leads to memories that are more persistent, and therefore are consistent with a longer retention period (Milner et al., 1998). However, if extinction is also a learning mechanism, then Tg-*ncs-1* worms need more time to react to the absence of one conditioning stimulus (i.e. food) that is likely to be linked to the level of [Ca²⁺]ᵢ signaling (see Figures 5 and 6).
The loss-of-function and mosaic rescue data obtained in accordance with the present invention clearly demonstrate that the presence and amount of the calcium sensor NCS-1 in AIY neurons plays a central role in influencing Ca²⁺-dependent associative learning in *C. elegans* as demonstrated by its direct regulatory effects on IT behavior. Furthermore, it could be show that calcium signaling or binding by NCS-1 is critical for this activity, and that the NCS-1 signaling pathway is essential for performing IT behavior. As shown on the schematic diagram (Figure 6), NCS-1 could have a presynaptic role at the AIY interneuron synapses with AIZ and RIA, or a post-synaptic function at the AFD/AIY synapses. The presynaptic activity of NCS-1 is supported by preliminary data indicating that increased levels of NCS-1 in the mouse hippocampus enhance LTP via a presynaptic facilitation. Similarly, the increase of IT behavior observed when NCS-1 is overexpressed (Tg-*ncs-1* animals) may reflect a state where the AIY pre-synaptic terminals are maximally stimulated. The observation of a presynaptic effect on overexpression of NCS-1 at the neuromuscular junction of mice, flies and frogs (Olafsson et al., 1995; Rivosecchi et al., 1994) supports this hypothesis and suggests a conserved function for NCS-1 through evolution.
The AIY interneuron could probably serve as an integrator of food and temperature inputs in the form of Ca²⁺ signals provided by the AFD and surrounding cells. These signals, detected by the neuronal calcium sensor NCS-1, could be transmitted to further downstream targets such as 3':5'-cyclic nucleotide phosphodiesterase, calcineurin, nitric oxide synthase, potassium channels, or phosphatidylinositol 4-OH kinase via mechanisms that could influence AIY synaptic strength. Ca²⁺-signaling via NCS-1 therefore defines a novel pathway for the regulation of synaptic efficacy.
Together, these thermotaxis enhanced or deficient NCS-1 strains provide valuable tools to study synaptic plasticity at the molecular, cellular and network levels using live animals, as well as a model that might well help to understand conserved functions such as long term memory and associative learning across species.

Usually, said transgenic non-human animal displaying a reduced level of neuron-specific calcium sensor-1 (NCS-1) activity comprises at least one mutant allele of the NCS-1 encoding gene or a corresponding trans-dominant allele of a different gene. Preferably, said transgenic non-human animal is a *ncs*-1 knock-out animal.

In a particularly preferred embodiment the method of the present invention said transgenic non-human animal is *C. elegans* and said behavior is isothermal tracking (IT). As described above and illustrated by the examples, the present invention for the first time provides the functional assay that is able to directly link the molecular action of a modulator of NCS-1 activity with a phenotypic response of a test animal. Since *C. elegans* is well characterized, is easy to handle and culture condition and other factors can be easily controlled, this test animal is particular suited for high through put screening; see for example Link et al., Therapeutic target discovery using C. elegans. Pharmacogenomics 1 (2000), 203-218.

The compounds which can be tested and identified according to a method of the invention may be expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). Furthermore, genes encoding a putative regulator of NCS-1 protein and/or which excert their effects up- or downstream the NCS-1 protein may be identified using, for example, insertion mutagenesis using, for example, gene targeting vectors known in the art. Likewise, the methods of the invention include ENU mutagenesis and suppressor screens that could be preformed to find regulator of NCS-1 dysfunction, hyperfunction, targets, or signaling pathways. Said compounds can also be functional derivatives or analogues of known ligands, for example Ca²⁺. Such useful compounds can also be for example transacting factors which bind to the NCS-1 protein or regulatory sequences of the NCS-1 gene.

The compounds isolated by the above methods can also serve as lead compounds for the development of analog compounds. The analogs should have a stabilized electronic configuration and molecular conformation that allows key functional groups to be presented to the NCS-1 protein or its receptor in substantially the same way as the lead compound. In particular, the analog compounds have spatial electronic properties which are comparable to the binding region, but can be smaller molecules than the lead compound, frequently having a molecular weight below about 2 kD and preferably below about 1 kD. Identification of analog compounds can be performed through use of techniques such as self-consistent field (SCF) analysis, configuration interaction (CI) analysis, and normal mode dynamics analysis. Computer programs for implementing these techniques are available; e.g., Rein, Computer-Assisted Modeling of Receptor-Ligand Interactions (Alan Liss, New York, 1989). Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art; see also supra. Furthermore, peptidomimetics and/or computer aided design of appropriate derivatives and analogues can be used, for example, according to the methods described above. Methods for the lead generation in drug discovery also include using proteins and detection methods such as mass spectrometry (Cheng et al. J. Am. Chem. Soc. 117 (1995), 8859-8860) and some nuclear magnetic resonance (NMR) methods (Fejzo et al., Chem. Biol. 6 (1999), 755-769; Lin et al., J. Org. Chem. 62 (1997), 8930-8931).

The newly identified drug obtained by a method of the present invention, i.e. an antagonistlinhibitor or agonist/activator can be used for the preparation of a pharmaceutical composition for the treatment of a NCS-1 protein mediated or related disorder. In accordance with this, the present invention also relates to a method of producing a drug comprising the steps of any one of the above-described methods; and
(i) synthesizing the drug candidate identified in step (b) or obtained in step (c) or an analog or derivative thereof in an amount sufficient to provide said drug in a therapeutically effective amount to a subject; and/or
(ii) combining the drug candidate identified in step (b) or obtained in step (c) or an analog or derivative thereof with a pharmaceutically acceptable carrier

Once a drug has been selected in accordance with any one of the above-described methods of the present invention, the drug or a pro-drug thereof can be synthesized in a therapeutically effective amount. As used herein, the term "therapeutically effective amount" means the total amount of the drug or pro-drug that is sufficient to show a meaningful patient benefit, i.e., treatment, healing, prevention or amelioration of a condition related to an NCS-1 protein, or an increase in rate of treatment, healing, prevention or amelioration of such conditions. In addition or alternatively, in particular with respect to pre-clinical testing of the drug the term "therapeutically effective amount" includes the total amount of the drug or pro-drug that is sufficient to elicit a physiological response, preferably upon its binding to its target NCS-1 protein, in an non-human animal test, preferably in a *C. elegans* assay such as described herein.

Drugs or pro-drugs after their *in vivo* administration are metabolized in order to be eliminated either by excretion or by metabolism to one or more active or inactive metabolites (Meyer, J. Pharmacokinet. Biopharm. 24 (1996), 449-459). Thus, rather than using the actual compound or drug identified and obtained in accordance with the methods of the present invention a corresponding formulation as a pro-drug can be used which is converted into its active in the patient. Precautionary measures that may be taken for the application of pro-drugs and drugs are described in the literature; see, for review, Ozama, J. Toxicol. Sci. 21 (1996), 323-329.

The invention further relates to a method of producing a pharmaceutical composition comprising an antagonist or inhibitor as described above comprising the steps of (a) modifying an inhibitor identified by the method of the invention as a lead compound to achieve (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmakinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carbon acids, or (iii) esterification of hydroxyl groups to, e.g. phosphates, pyrophosphates or sulfates or hemi succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiff's bases, oximes, acetales, ketales, enolesters, oxazolidines, thiozolidines or combinations thereof; and (b) formulating the product of said modification with a pharmaceutically acceptable carrier.

The various steps recited above are generally known in the art. They include or rely on quantitative structure-action relationship (QSAR) analyses (Kubinyi, 1993), combinatorial biochemistry, classical chemistry and others (see, for example, Holzgrabe and Bechtold, 2000).

As mentioned above, the present invention provides convenient assays, preferably in vivo assays for identifying and obtaining drugs capable of modulating NCS-1 activity, thereby being useful as a therapeutic agent for the treatment of diseases related to NCS-1 activity such as CNS disorders including Schizophrenia, Parkinson's Disease, Alzheimer's Disease, and other behavioral disorders. Thus, the present invention provides therapeutic agents which mode of action is different from compounds previously used for the treatment of the mentioned disorders. In accordance with this, the present invention provides also a use for compounds which have been known in the art, properly also known to be able to modulate NCS-1 activity but which hitherto have not been suggested for medical use because of the lack of knowledge of phenotypic responses of an organism evoked by NCS-1 activity or the lack of it.

In a further embodiment the present invention relates to a pharmaceutical composition comprising a drug or drug candidate identified or obtained by the method of the invention or a racemate, enantiomer, diastereomer, tautomer, mixture of diastereomers or pharmaceutically acceptable salt of any one those, wherein said drug or drug candidate is a modulator of NCS-1 activity. Preferably, said drug facilitates or interferes with calcium binding of NCS-1 or changes conformation of NCS-1.

The present invention also relates to transgenic non-human animals displaying a reduced level of neuron-specific calcium sensor-1 (NCS-1) activity comprises at least one mutant allele of the NCS-1 encoding gene or a corresponding trans-dominant allele of a different gene. Preferably, said transgenic non-human animal is a *ncs*-1 knock-out animal. In a particularly preferred embodiment, said transgenic non-human animal is *C. elegans* and said behavior is isothermal tracking (IT).

A method for the production of a transgenic non-human animal, for example transgenic mouse, comprises introduction of a NCS-1 polynucleotide or targeting vector into a germ cell, an embryonic cell, stem cell or an egg or a cell derived therefrom. The non-human animal can be used in accordance with a screening method of the invention described herein. Production of transgenic embryos and screening of those can be performed, e.g., as described by A. L. Joyner Ed., Gene Targeting, A Practical Approach (1993), Oxford University Press. The DNA of the embryonal membranes of embryos can be analyzed using, e.g., Southern blots with an appropriate probe; see supra. The invention also relates to transgenic non-human animals such as transgenic mouse, rats, hamsters, dogs, monkeys, rabbits, pigs, *C. elegans* and fish such as Torpedo fish comprising a NCS-1 gene. Preferably, said transgenic non-human animal is *C. elegans* such as a mutant animal described in the examples. Preferably, the transgenic non-human animal comprises at least one inactivated or suppressed wild type allele of the corresponding NCS-1 encoding gene; see supra. This embodiment allows for example the study of the interaction of various mutant forms of NCS-1 polypeptides on the onset of the clinical symtoms a of disease related to disorders in the calcium signaling pathway. All the applications that have been herein before discussed with regard to a transgenic animal also apply to animals carrying two, three or more transgenes for example encoding calmodulin. It might be also desirable to inactivate NCS-1 protein expression or function at a certain stage of development and/or life of the transgenic animal. This can be achieved by using, for example, tissue specific, developmental and/or cell regulated and/or inducible promoters which drive the expression of, e.g., an antisense or ribozyme directed against the RNA transcript encoding the NCS-1 encoding RNA; see also supra. A suitable inducible system is for example tetracycline-regulated gene expression as described, e.g., by Gossen and Bujard (Proc. Natl. Acad. Sci. 89 USA (1992), 5547-5551) and Gossen et al. (Trends Biotech. 12 (1994), 58-62). Similar, the expression of the mutant NCS-1 protein may be controlled by such regulatory elements.

Furthermore, the invention also relates to a transgenic, preferably eukaryotic cell which contains (preferably stably integrated into its genome) a NCS-1 nucleic acid molecule or part thereof, wherein the transcription and/or expression of the nucleic acid molecule or part thereof leads to reduction of the synthesis of a NCS-1 protein. In a preferred embodiment, the reduction is achieved by an anti-sense, sense, ribozyme, co-suppression and/or dominant mutant effect. "Antisense" and "antisense nucleotides" means DNA or RNA constructs which block the expression of the naturally occurring gene product.
Techniques how to achieve this are well known to the person skilled in the art. These include, for example, the expression of antisense-RNA, ribozymes, of molecules which combine antisense and ribozyme functions and/or of molecules which provide for a co-suppression effect; see also supra. When using the antisense approach for reduction of the amount of NCS-1 proteins in cells, the nucleic acid molecule encoding the antisense-RNA is preferably of homologous origin with respect to the animal species used for transformation. However, it is also possible to use nucleic acid molecules which display a high degree of homology to endogenously occurring nucleic acid molecules encoding a NCS-1 protein. In this case the homology is preferably higher than 80%, particularly higher than 90% and still more preferably higher than 95%. The reduction of the synthesis of NCS-1 protein in the transgenic eukaryotic cells can result in an alteration in, e.g., calcium signaling. In transgenic animals comprising such cells this can lead to various physiological, developmental and/or morphological changes, preferably to a diminution of cognitive functions such as learning, memory, attention, or hyperactivity. Such behavioral assessments can be performed in rodent and non-rodent species.

Thus, the present invention also relates to transgenic non-human animals comprising the above-described transgenic cells. These may show, for example, a deficiency or other alteration in calcium signaling compared to wild type animals due to the stable or transient presence of a foreign DNA resulting in at least one of the following features:
(a) disruption of (an) endogenous gene(s) encoding NCS-1;
(b) expression of at least on antisense RNA and/or ribozyme against a transcript comprising a NCS-1 polynucleotide;
(c) expression of a sense and/or non-translatable mRNA of an NCS-1 polynucleotide;
(d) expression of an anti-NCS-1 antibody;
(e) incorporation of a functional or non-functional copy of a regulatory sequence of the NCS-1 gene; or
(f) incorporation of a recombinant DNA molecule or vector comprising any one of the above-described polynucleotides or nucleic acid molecules.

With the NCS-1 polypeptides, their encoding polynucleotides and corresponding vectors, it is now possible to study *in vivo* and *in vitro* the efficiency of drugs in relation to particular mutations in NCS-1 proteins of a patient and the affected phenotype. Furthermore, mutant forms of NCS-1 polypeptides can be used to determine the pharmacological profile of drugs and for the identification and preparation of further drugs which may be effective for the treatment of disorders related to the calcium signaling, in particular for the amelioration of certain phenotypes caused by the respective mutations, in the NCS-1 encoding gene.

Over the past 20 years, genetic heterogeneity has been increasingly recognized as a significant source of variation in drug response. Many scientific communications (Meyer, Ann. Rev. Pharmacol. Toxicol. 37 (1997), 269-296 and West, J. Clin. Pharmacol. 37 (1997), 635-648) have clearly shown that some drugs work better or may even be highly toxic in some patients than in others and that these variations in patient's responses to drugs can be related to molecular basis. This "pharmacogenomic" concept spots correlations between responses to drugs and genetic profiles of patient's (Marshall, Nature Biotechnology, 15 (1997), 954-957; Marshall, Nature Biotechnology, 15 (1997), 1249-1252).
In this context of population variability with regard to drug therapy, pharmacogenomics has been proposed as a tool useful in the identification and selection of patients which can respond to a particular drug without side effects. This identification/selection can be based upon molecular diagnosis of genetic polymorphisms by genotyping DNA from leukocytes in the blood of patient, for example, and characterization of disease (Bertz, Clin. Pharmacokinet. 32 (1997), 210-256; Engel, J. Chromatogra. B. Biomed. Appl. 678 (1996), 93-103). For the founders of health care, such as health maintenance organizations in the US and government public health services in many European countries, this pharmacogenomics approach can represent a way of both improving health care and reducing overheads because there is a large cost to unnecessary drugs, ineffective drugs and drugs with side effects.

Hence another object of the present invention concerns the pharmacogenomic selection of drugs and prodrugs for patients suffering from CNS disorders such as those described above and which are possible candidates to drug therapy. Thus, the findings of the present invention provide the options of development of new drugs for the pharmalogical intervention with the aim of restituing the function of genetically modified NCS-1 proteins. Also a gene therapeutical approach can be envisaged with the aid of the present invention.

In accordance with the above, the present invention also relates to the use of a neuron-specific calcium sensor-1 (NCS-1) or a biologically active fragment thereof, a nucleic acid molecule encoding NCS-1 or nucleic acid molecule of at least 15 nucleotides in length hybridizing to a *ncs*-1 gene, an anti-NCS-1 antibody, a cell as described above or of an NCS-1 activity assay for a method of obtaining, identifying and/or profiling a drug candidate for therapy of a CNS disorder or for modulating cognition of subject.

Nucleotide sequences that are complementary to the NCS-1 encoding gene sequence can be synthesized for antisense therapy. These antisense molecules may be DNA, stable derivatives of DNA such as phosphorothioates or methylphosphonates, RNA, stable derivatives of RNA such as 2'-*O*-alkylRNA, or other NCS-1 antisense oligonucleotide mimetics. NCS-1 antisense molecules may be introduced into cells by microinjection, liposome encapsulation or by expression from vectors harboring the antisense sequence. NCS-1 antisense therapy may be particularly useful for the treatment of diseases where it is beneficial to reduce NCS-1 activity.
NCS-1 gene therapy may be used to introduce NCS-1 into the cells of target organisms. The NCS-1 gene can be ligated into viral vectors that mediate transfer of the NCS-1 DNA by infection of recipient host cells. Suitable viral vectors include retrovirus, adenovirus, adeno-associated virus, herpes virus, vaccinia virus, polio virus and the like. Alternatively, NCS-1 DNA can be transferred into cells for gene therapy by non-viral techniques including receptor-mediated targeted DNA transfer using ligand-DNA conjugates or adenovirus-ligand-DNA conjugates, lipofection membrane fusion or direct microinjection. These procedures and variations thereof are suitable for *ex vivo* as well as *in vivo* NCS-1 gene therapy. NCS-1 gene therapy may be particularly useful for the treatment of diseases where it is beneficial to elevate NCS-1 activity. Protocols for molecular methodology of gene therapy suitable for use with the NCS-1 gene is described in Gene Therapy Protocols, edited by Paul D. Robbins, Human press, Totawa NJ, 1996.

Pharmaceutically useful compositions such as described herein-before, comprising NCS-1 DNA, NCS-1 RNA, or NCS-1 protein, or modulators of NCS-1 activity, i.e. activator/agonist or inhibitor/antagonist, may be formulated according to known methods such as by the admixture of a pharmaceutically acceptable carrier. Examples of such carriers and methods of formulation may be found in Remington's Pharmaceutical Sciences. To form a pharmaceutically acceptable composition suitable for effective administration, such compositions will contain an effective amount of the protein, DNA, RNA, or modulator.
Therapeutic or diagnostic compositions of the invention are administered to an individual in amounts sufficient to treat or diagnose disorders in which modulation of NCS-1-related activity is indicated. The effective amount may vary according to a variety of factors such as the individual's condition, weight, sex and age. Other factors include the mode of administration. The pharmaceutical compositions may be provided to the individual by a variety of routes such as subcutaneous, topical, oral and intramuscular.
The term "chemical derivative" describes a molecule that contains additional chemical moieties that are not normally a part of the base molecule. Such moieties may improve the solubility, half-life, absorption, etc. of the base molecule. Alternatively the moieties may attenuate undesirable side effects of the base molecule or decrease the toxicity of the base molecule. Examples of such moieties are described in a variety of texts, such as Remington's Pharmaceutical Sciences.
Compounds identified according to the methods disclosed herein may be used alone at appropriate dosages defined by routine testing in order to obtain optimal inhibition of the NCS-1 receptor or its activity while minimizing any potential toxicity. In addition, coadministration or sequential administration of other agents may be desirable.

A therapeutically effective dose refers to that amount of protein or its antibodies, agonists, activators, antagonists, or inhibitors which ameliorate the symptoms or condition. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The present invention also has the objective of providing suitable topical, oral, systemic and parenteral pharmaceutical formulations for use in the novel methods of treatment of the present invention. The compositions containing compounds or modulators identified according to this invention as the active ingredient for use in the modulation of NCS-1 can be administered in a wide variety of therapeutic dosage forms in conventional vehicles for administration. For example, the compounds or modulators can be administered in such oral dosage forms as tablets, capsules (each including timed release and sustained release formulations), pills, powders, granules, elixirs, tinctures, solutions, suspensions, syrups and emulsions, or by injection. Likewise, they may also be administered in intravenous (both bolus and infusion), intraperitoneal, subcutaneous, topical with or without occlusion, or intramuscular form, all using forms well known to those of ordinary skill in the pharmaceutical arts. An effective but non-toxic amount of the compound desired can be employed as a NCS-1 modulating agent.
The daily dosage of the products may be varied over a wide range from 0.01 to 1,000 mg per patient, per day. For oral administration, the compositions are preferably provided in the form of scored or unscored tablets containing 0.01, 0.05, 0.1, 0.5, 1.0, 2.5, 5.0, 10.0, 15.0, 25.0, and 50.0 milligrams of the active ingredient for the symptomatic adjustment of the dosage to the patient to be treated. An effective amount of the drug is ordinarily supplied at a dosage level of from about 0.0001 mg/kg to about 100 mg/kg of body weight per day. The range is more particularly from about 0.001 mg/kg to 10 mg/kg of body weight per day. The dosages of the NCS-1 modulators are adjusted when combined to achieve desired effects. On the other hand, dosages of these various agents may be independently optimized and combined to achieve a synergistic result wherein the pathology is reduced more than it would be if either agent were used alone.
Advantageously, compounds or modulators of the present invention may be administered in a single daily dose, or the total daily dosage may be administered in divided doses of two, three or four times daily. Furthermore, compounds or modulators for the present invention can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen.
For combination treatment with more than one active agent, where the active agents are in separate dosage formulations, the active agents can be administered concurrently, or they each can be administered at separately staggered times.

The dosage regimen utilizing the compounds or modulators of the present invention is selected in accordance with a variety of factors including type, species, age, weight, sex and medical condition of the patient; the severity of the condition to be treated; the route of administration; the renal and hepatic function of the patient; and the particular compound thereof employed. A physician or veterinarian of ordinary skill can readily determine and prescribe the effective amount of the drug required to prevent, counter or arrest the progress of the condition. Optimal precision in achieving concentrations of drug within the range that yields efficacy without toxicity requires a regimen based on the kinetics of the drug's availability to target sites. This involves a consideration of the distribution, equilibrium, and elimination of a drug.
In the methods of the present invention, the compounds or modulators herein described in detail can form the active ingredient, and are typically administered in admixture with suitable pharmaceutical diluents, excipients or carriers (collectively referred to herein as "carrier" materials) suitably selected with respect to the intended form of administration, that is, oral tablets, capsules, elixirs, syrups and the like, and consistent with conventional pharmaceutical practices.
For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include, without limitation, sodium oleate, sodium stearate, magnesium stearate, sodium benzoate, sodium acetate, sodium chloride and the like. Disintegrators include, without limitation, starch, methyl cellulose, agar, bentonite, xanthan gum and the like.
For liquid forms the active drug component can be combined in suitably flavored suspending or dispersing agents such as the synthetic and natural gums, for example, tragacanth, acacia, methyl-cellulose and the like. Other dispersing agents that may be employed include glycerin and the like. For parenteral administration, sterile suspensions and solutions are desired. Isotonic preparations, which generally contain suitable preservatives, are employed when intravenous administration is desired.

Topical preparations containing the active drug component can be admixed with a variety of carrier materials well known in the art, such as, e.g., alcohols, aloe vera gel, allantoin, glycerine, vitamin A and E oils, mineral oil, PPG2 myristyl propionate, and the like, to form, e.g., alcoholic solutions, topical cleansers, cleansing creams, skin gels, skin lotions, and shampoos in cream or gel formulations.
The compounds or modulators of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.
Compounds of the present invention may also be delivered by the use of monoclonal antibodies as individual carriers to which the compound molecules are coupled. The compounds or modulators of the present invention may also be coupled with soluble polymers as targetable drug carriers. Such polymers can include polyvinyl-pyrrolidone, pyran copolymer, polyhydroxypropylmethacryl-amidephenol, polyhydroxy-ethylaspartamidephenol, or polyethyl-eneoxidepolylysine substituted with palmitoyl residues. Furthermore, the compounds or modulators of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydro-pyrans, polycyanoacrylates and cross-linked or amphipathic block copolymers of hydrogels.
For oral administration, the compounds or modulators may be administered in capsule, tablet, or bolus form or alternatively they can be mixed in the animals feed. The capsules, tablets, and boluses are comprised of the active ingredient in combination with an appropriate carrier vehicle such as starch, talc, magnesium stearate, or di-calcium phosphate. These unit dosage forms are prepared by intimately mixing the active ingredient with suitable finely-powdered inert ingredients including diluents, fillers, disintegrating agents, and/or binders such that a uniform mixture is obtained. An inert ingredient is one that will not react with the compounds or modulators and which is non-toxic to the animal being treated. Suitable inert ingredients include starch, lactose, talc, magnesium stearate, vegetable gums and oils, and the like. These formulations may contain a widely variable amount of the active and inactive ingredients depending on numerous factors such as the size and type of the animal species to be treated and the type and severity of the disorder. The active ingredient may also be administered as an additive to the feed by simply mixing the compound with the feedstuff or by applying the compound to the surface of the feed. Alternatively the active ingredient may be mixed with an inert carrier and the resulting composition may then either be mixed with the feed or fed directly to the animal. Suitable inert carriers include corn meal, citrus meal, fermentation residues, soya grits, dried grains and the like. The active ingredients are intimately mixed with these inert carriers by grinding, stirring, milling, or tumbling such that the final composition contains from 0.001 to 5% by weight of the active ingredient.
The compounds or modulators may alternatively be administered parenterally via injection of a formulation consisting of the active ingredient dissolved in an inert liquid carrier. Injection may be either intramuscular, intraruminal, intratracheal, or subcutaneous. The injectable formulation consists of the active ingredient mixed with an appropriate inert liquid carrier. Acceptable liquid carriers include the vegetable oils such as peanut oil, cotton seed oil, sesame oil and the like as well as organic solvents such as solketal, glycerol formal and the like. As an alternative, aqueous parenteral formulations may also be used. The vegetable oils are the preferred liquid carriers. The formulations are prepared by dissolving or suspending the active ingredient in the liquid carrier such that the final formulation contains from 0.005 to 10% by weight of the active ingredient.
Topical application of the compounds or modulators is possible through the use of a liquid drench or a shampoo containing the instant compounds or modulators as an aqueous solution or suspension. These formulations generally contain a suspending agent such as bentonite and normally will also contain an antifoaming agent. Formulations containing from 0.005 to 10% by weight of the active ingredient are acceptable. Preferred formulations are those containing from 0.01 to 5% by weight of the instant compounds or modulators.

The present invention also relates to a method of treating a patient in need of such treatment for a disorder which is mediated by neuron-specific calcium sensor-1 (NCS-1), comprising administration of a drug or drug candidate identified or obtained in any one of the above-described methods. In addition, the present invention relates to a method for treating a CNS disorder in a subject or improving cognition of a subject, which method comprises administering to the subject an effective amount of a therapeutic agent to increase the level and/or activity of NCS-1, so as to improve or restore calcium signaling in the subject.

In a preferred embodiment of the uses and methods of the present invention said disorder is or is related to Alzheimer's disease, Parkinson's disease, age-associated cognition deficits, major depression, bipolar disorder, anxiety disorders, appetite disorders, sleep disorders, insomnia, attention deficit hyperactivity disorder or memory loss or a learning deficiency.

In a further aspect the present invention relates to a method of diagnosing a pathological condition or a susceptibility to a pathological condition in a subject related to a CNS disorder:
(a) determining the presence or absence of a mutation in the polynucleotide encoding neuron-specific calcium sensor-1 (NCS-1); and
(b) diagnosing a pathological condition or a susceptibility to a pathological condition based on the presence or absence of said mutation.

In another embodiment the present invention relates to a method of diagnosing a pathological condition or a susceptibility to a pathological condition in a subject related to a CNS disorder comprising:
(a) determining the presence or amount of expression of a neuron-specific calcium sensor-1 (NCS-1) polypeptide in a biological sample; and
(b) diagnosing a pathological condition or a susceptibility to a pathological condition based on the presence or amount of expression of the polypeptide.

In these embodiments, the NCS-1 polynucleotides, nucleic acid molecules, (poly)peptide, antibodies or compounds identified above are preferably detectably labeled. A variety of techniques are available for labeling biomolecules, are well known to the person skilled in the art and are considered to be within the scope of the present invention. Such techniques are, e.g., described in Tijssen, "Practice and theory of enzyme immuno assays", Burden, RH and von Knippenburg (Eds), Volume 15 (1985), "Basic methods in molecular biology"; Davis LG, Dibmer MD; Battey Elsevier (1990), Mayer et al., (Eds) "Immunochemical methods in cell and molecular biology" Academic Press, London (1987), or in the series "Methods in Enzymology", Academic Press, Inc. There are many different labels and methods of labeling known to those of ordinary skill in the art. Commonly used labels comprise, inter alia, fluorochromes (like fluorescein, rhodamine, Texas Red, etc.), enzymes (like horse radish peroxidase, β-galactosidase, alkaline phosphatase), radioactive isotopes (like ³²P or ¹²⁵I), biotin, digoxygenin, colloidal metals, chemi- or bioluminescent compounds (like dioxetanes, luminol or acridiniums). Labeling procedures, like covalent coupling of enzymes or biotinyl groups, iodinations, phosphorylations, biotinylations, random priming, nick-translations, tailing (using terminal transferases) are well known in the art. Detection methods comprise, but are not limited to, autoradiography, fluorescence microscopy, direct and indirect enzymatic reactions, etc.
In addition, the above-described compounds etc. may be attached to a solid phase. Solid phases are known to those in the art and may comprise polystyrene beads, latex beads, magnetic beads, colloid metal particles, glass and/or silicon chips and surfaces, nitrocellulose strips, membranes, sheets, animal red blood cells, or red blood cell ghosts, duracytes and the walls of wells of a reaction tray, plastic tubes or other test tubes. Suitable methods of immobilizing NCS-1 nucleic acids, (poly)peptides, proteins, antibodies, etc. on solid phases include but are not limited to ionic, hydrophobic, covalent interactions and the like. The solid phase can retain one or more additional receptor(s) which has/have the ability to attract and immobilize the region as defined above. This receptor can comprise a charged substance that is oppositely charged with respect to the reagent itself or to a charged substance conjugated to the capture reagent or the receptor can be any specific binding partner which is immobilized upon (attached to) the solid phase and which is able to immobilize the reagent as defined above.
Commonly used detection assays can comprise radioisotopic or non-radioisotopic methods. These comprise, inter alia, RIA (Radioisotopic Assay) and IRMA (Immune Radioimmunometric Assay), EIA (Enzym Immuno Assay), ELISA (Enzyme Linked Immuno Assay), FIA (Fluorescent Immuno Assay), and CLIA (Chemioluminescent Immune Assay). Other detection methods that are used in the art are those that do not utilize tracer molecules. One prototype of these methods is the agglutination assay, based on the property of a given molecule to bridge at least two particles.

For diagnosis and quantification of (poly)peptides, polynucleotides, etc. in clinical and/or scientific specimens, a variety of immunological methods, as described above as well as molecular biological methods, like nucleic acid hybridization assays, PCR assays or DNA Enzyme Immunoassays (Mantero et al., Clinical Chemistry 37 (1991), 422-429) have been developed and are well known in the art. In this context, it should be noted that the NCS-1 nucleic acid molecules may also comprise PNAs, modified DNA analogs containing amide backbone linkages. Such PNAs are useful, inter alia, as probes for DNA/RNA hybridization.

The above-described compositions may be used for methods for detecting expression of a NCS-1 polynucleotide by detecting the presence of mRNA coding for a NCS-1 (poly)peptide which comprises, for example, obtaining mRNA from cells of a subject and contacting the mRNA so obtained with a probe/primer comprising a nucleic acid molecule capable of specifically hybridizing with a NCS-1 polynucleotide under suitable hybridization conditions, and detecting the presence of mRNA hybridized to the probe/primer. Further diagnostic methods leading to the detection of nucleic acid molecules in a sample comprise, e.g., polymerase chain reaction (PCR), ligase chain reaction (LCR), Southern blotting in combination with nucleic acid hybridization, comparative genome hybridization (CGH) or representative difference analysis (RDA). These methods for assaying for the presence of nucleic acid molecules are known in the art and can be carried out without any undue experimentation.

Furthermore, the invention comprises methods of detecting the presence of a NCS-1 protein in a sample, for example, a cell sample, which comprises obtaining a cell sample from a subject, contacting said sample with one of the aforementioned antibodies under conditions permitting binding of the antibody to the NCS-1 protein, and detecting the presence of the antibody so bound, for example, using immuno assay techniques such as radioimmunoassay or enzymeimmunoassay. Furthermore, one skilled in the art may specifically detect and distinguish polypeptides which are functional NCS-1 proteins from mutated forms which have lost or altered their NCS-1 activity by using an antibody which either specifically recognizes a (poly)peptide which has NCS-1 activity but does not recognize an inactive form thereof or which specifically recognizes an inactive form but not the corresponding polypeptide having NCS-1 activity.

The invention also encompasses a method for diagnosing in a subject a predisposition to a CNS disorder associated with the expression of a NCS-1 allele which comprises isolating DNA from victims of the disorder associated with the under-or over-expression of a NCS-1 protein or a mutant form thereof; digesting the isolated DNA with at least one restriction enzyme; electrophoretically separating the resulting DNA fragments on a sizing gel; contacting the resulting gel with a nucleic acid probe as described above capable of specifically hybridizing to DNA encoding a NCS-1 protein and labeled with a detectable marker; detecting labeled bands on the gel which have hybridized to the labeled probe to create a band pattern specific to the DNA of victims of the disorder associated with the expression of a NCS-1 protein; preparing the subject's DNA according to the above-mentioned steps to produce detectable labeled bands on a gel; and comparing the band pattern specific to the DNA of victims of the disorder associated with the expression of a NCS-1 protein and the subject's DNA to determine whether the patterns are the same or different and to diagnose thereby predisposition to the disorder if the patterns are the same. The detectable markers of the present invention may be labeled with commonly employed radioactive labels, such as, for example, ³²P and ³⁵S, although other labels such as biotin or mercury as well as those described above may be employed as well. Various methods well-known to the person skilled in the art may be used to label the detectable markers. For example, DNA sequences and RNA sequences may be labeled with ³²P or ³⁵S using the random primer method. Once a suitable detectable marker has been obtained, various methods well-known to the person skilled in the art may be employed for contacting the detectable marker with the sample of interest. For example, DNA-DNA, RNA-RNA and DNA-RNA hybridizations may be performed using standard procedures. Various methods for the detection of nucleic acids are well-known in the art, e.g., Southern and northern blotting, PCR, primer extension and the like. Suitable further DNA amplification techniques are known in the art and comprise, inter alia, Ligase Chain reaction, Strand Displacement Amplification, Nucleic Acid Sequence based Amplification (NASBA), or Q-beta replicase.
Furthermore, the mRNA, cRNA, cDNA or genomic DNA obtained from the subject may be sequenced to identify mutations which may be characteristic fingerprints of NCS-1 mutations in CNS disorders such as described above associated with the expression of NCS-1 or mutated versions thereof. The present invention further comprises methods, wherein such a fingerprint may be generated by RFLPs or AFLP of DNA or RNA obtained from the subject, optionally the DNA or RNA may be amplified prior to analysis, the methods of which are well known in the art. RNA fingerprints may be performed by, for example, digesting an RNA sample obtained from the subject with a suitable RNA-Enzyme, for example RNase T₁, RNase T₂ or the like or a ribozyme and, for example, electrophoretically separating and detecting the RNA fragments on PAGE as described above. Preferably, hybridization (and subsequent washing) is effected under stringent conditions; see, e.g., Sambrook et al., loc. cit and supra.
Furthermore, the present invention relates to a method as described above wherein said sample is or is derived from hair, blood, serum, sputum, feces or another body fluid. The sample to be analyzed may be treated such as to extract, inter alia, nucleic acid molecules, (poly)peptides, or antibodies.

The present invention also relates to kit compositions containing NCS-1 specific reagents such as those described herein-before. Kits containing NCS-1 DNA or RNA, antibodies to NCS-1, or NCS-1 protein may be prepared. Such kits are used to detect DNA which hybridizes to NCS-1 DNA or to detect the presence of NCS-1 protein or peptide fragments in a sample. Such characterization is useful for a variety of purposes including but not limited to forensic analyses, diagnostic applications, and epidemiological studies in accordance with the above-described methods of the present invention.
The recombinant NCS-1 proteins, DNA molecules, RNA molecules and antibodies lend themselves to the formulation of kits suitable for the detection and typing of NCS-1. Such a kit would typically comprise a compartmentalized carrier suitable to hold in close confinement at least one container. The carrier would further comprise reagents such as recombinant NCS-1 protein or anti-NCS-1 antibodies suitable for detecting NCS-1. The carrier may also contain a means for detection such as labeled antigen or enzyme substrates or the like.

These and other embodiments are disclosed and encompassed by the description and Examples of the present invention. Further literature concerning any one of the antibodies, methods, uses and compounds to be employed in accordance with the present invention may be retrieved from public libraries and databases, using for example electronic devices. For example the public database "Medline" may be utilized which is available on the Internet, for example under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools.html, http://www.tigr.org/, are known to the person skilled in the art and can also be obtained using, e.g., http://www.lycos.com. An overview of patent information in biotechnology and a survey of relevant sources of patent information useful for retrospective searching and for current awareness is given in Berks, TIBTECH 12 (1994), 352-364.

This disclosure may best be understood in conjunction with the accompanying drawings, incorporated herein by references. Furthermore, a better understanding of the present invention and of its many advantages will be had from the following examples, given by way of illustration and are not intended as limiting.

Unless stated otherwise in the examples, all recombinant DNA techniques are performed according to protocols as described in Sambrook et al. (1989), Molecular Cloning : A Laboratory Manual. Cold Spring Harbor Laboratory Press, NY or in Volumes 1 and 2 of Ausubel et al. (1994), Current Protocols in Molecular Biology, Current Protocols. Standard materials and methods for plant molecular work are described in Plant Molecular Biology Labfase (1993) by R.D.D. Croy, jointly published by BIOS Scientific Publications Ltd (UK) and Blackwell Scientific Publications (UK). Site-directed mutagenesis was performed using the QuikChange Site-directed Mutagenesis kit (Stratagene). Wild-type *C. elegans* Bristol strain (N2) was obtained from the *Caenorhabditis* Genetics Center (funded by the NIH National Center for Research Resources).

The Figures show:

### Figure 1. Expression pattern of the ncs-1::GFP reporter gene

A) *ncs-1* gene expression is observed in amphid, phasmid, nerve ring, and ventral nerve cord of L1 stage animals as GFP staining. Scale bar: 100 µM.
B) *ncs-1* gene expression in adult head showing GFP staining in amphid dendrites. Scale bar: 10 µM

### Figure 2. Ce-NCS-1: from gene structure to calcium sensor

A) Physical maps of wild-type *Ce-ncs-1, ncs-1(pk242::Tc1),* and null *ncs-1(qa401te)* deletion genes. Black boxes represent exons 1-6, and the gray boxes the 5' and 3' untranslated regions of the *ncs-1* gene. Scale bar: 500 base pairs.
B) The NCS-1 protein contains 4 EF-hands (EF1-4), but the first binding site is degenerated and cannot bind Ca²⁺ (De Castro et al., 1995). The Asp positions D73, D109, and D157 are essential for calcium binding. Changing the three Asp residues (D*) into Ala inactivates Ca²⁺ binding (Putkey et al., 1989) (see below).
C) The loss-of-function (If) triple mutant was constructed by substituting the first Asp (D*) residue to an Ala of the three EF-hands EF2, 3 and 4.
D) *Ce*-NCS-1 is a calcium sensor. Calcium bound wild-type NCS-1 displayed a greater electrophoretic mobility than the apo form, whereas If-NCS-1 mobility was not affected by the presence (+Ca²⁺) or absence (+EGTA) of free calcium. It suggests that Ca²⁺ induces an allosteric change in the conformation and probably activity of *Ce*-NCS-1.

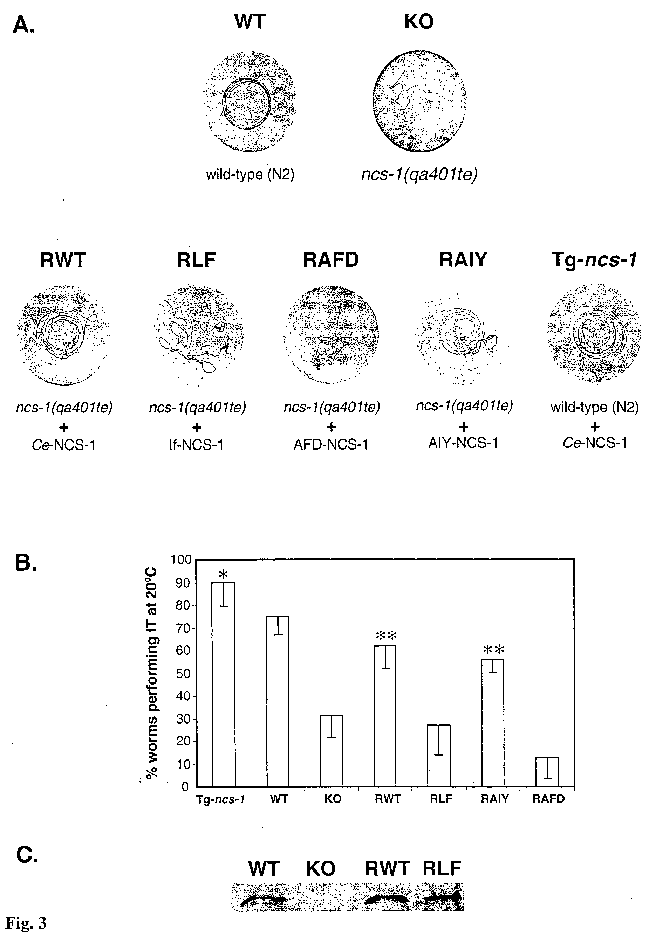

### Figure 3. Ca²⁺ signaling via NCS-1 in the AIY interneuron is essential for isothermal tracking behavior

A) Individual isothermal tracking (IT) records. Photographs of normal or disrupted isothermal behavior tracks of wild-type (WT), *ncs-1(qa401te)* knockout (KO), rescued *ncs-1(qa401te)* with wild-type *ncs-1* (RWT), or with loss-of-function *ncs-1* (RLF), or with AFD neuron specific promotor (RAFD) driving the expression of NCS-1, or with AIY neuron specific promotor (RAIY) driving the expression of NCS-1, and wild-type plus transgenic *ncs-1* (Tg*-ncs-1*) individual worms are shown. Thermotaxis assays were performed as described in (Mori and Ohshima, 1995).
B) Percentage (group performance) of worms performing IT behavior after overnight feeding at 20°C. Each data point represents 4-10 independent assays using approximately 10-20 animals per assay. At least 2-3 different lines were generated for each transgene construct. The chi-square distribution and T-test were used to determine the significance of IT behavior performance between the different strains. The P value (* ≤ 0.02) indicates a significant difference between Tg*-ncs-1* animals as compared to wild-type worms. The P values (** ≤ 0.002) represent significant differences of performance between KO animals and RWT or RAIY worms. For these experiments, standard deviations range from 7 to 14%. A trace is considered as isothermal if more than half of the trace length left on the agar surface by a single animal is circular or present an arc of circle near the isotherm of the growth temperature.
C) *Ce*-NCS-1 protein levels in the various WT, KO, RWT, RLF strains or lines. Western blot analysis using *Ce*-NCS-1 polyclonal antibodies and 80 µg of total protein extract reveals the presence of the NCS-1 calcium sensor in the wild-type strain (WT), in the NCS-1 rescued wild-type lines (RWT), and in the rescue loss-of-function lines (RLF). Note the absence of NCS-1 in the knockout strain (KO).

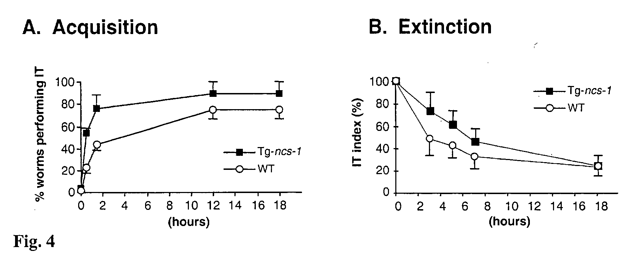

### Figure 4. Faster acquisition (learning) and longer retention (memory) for NCS-1 overexpressing worms

A) The acquisition of the association of food at a given temperature was determined for wild-type (WT) and overexpressing NCS-1 (Tg-*ncs-1*) worms by measuring the % of worms performing IT behavior at 20°C. Briefly, worms were grown on seeded plates at 25°C for at least 12 hours, then shifted individually to a seeded plate at 20°C for different time intervals. For both strains, the maximal levels of IT behavior (absolute values) were reached after pairing the conditioning stimuli for at least 12 hours. 50% of the maximum level was reached after 68 minutes for WT worms, and after only 28 minutes with Tg-*ncs-1* worms. As the half-maximal acquisition was scored instead of the relative IT index (see definition below), the experiment was internally controlled for increased performance for each strain.
B) The extinction of this association (food at 20°C) was determined for wild-type (WT) and overexpressing NCS-1 (Tg-*ncs-1*) worms. Briefly, worms were grown at 20°C in presence of food for at least 18 hours, washed at 20°C, and transferred to unseeded plate at 20°C for different time intervals. Normalized IT values (IT index) were used to correct for the increased performance of Tg*-ncs-1* worms after conditioning, and to only consider extinction of trained animals. 100% correspond to the mean performance achieved after 18 hours at 20°C (see Figure 4A for absolute values). Half maximal extinction was obtained after 3 hours with WT worms, whereas Tg*-ncs-1* worms had a prolonged retention, and reached half-maximal extinction after about 7 hours.

### Figure 5. Regulation of associative learning and memory by NCS-1

The schematic view indicates that the amount of NCS-1 directly regulates IT behavior. The absence of the neuronal calcium sensor-1 *(ncs-1* KO) impedes the majority of worms from performing isothermal tracking behavior, whereas its presence (WT) allows it. Overexpression of NCS-1 (Tg*-ncs-1*) enhances performance levels, accelerates learning, and produces a memory with slower extinction. Slower extinction might reflect increased responsiveness of the AIY integrative neurons to [Ca²⁺]ᵢ stimuli. The amount of NCS-1 in the AIY neurons and the strength of Ca²⁺ stimulation are linked together to modulate associative learning and memory in *C. elegans*. The dotted lines represent hypothetical IT responses.

### Figure 6. Model for a pre or postsynaptic role of the neuronal calcium sensor-1

NCS-1 is present in the AFD and AIY neurons, either at the dendritic or axonal terminals, and its function in the AIY neurons is essential for IT behavior. In this model, the AIY interneuron serves as an integrator of food and temperature inputs, and the NCS-1 calcium sensor transduces calcium signals and regulates synaptic strength between AIY/AIZ and AIY/RIA cells at the presynaptic location, or between AFD/AIY neurons in a postsynaptic location. The + or - sign indicates the presence of an excitatory or inhibitory synapse.

### Examples

### Example 1: Ce-ncs-1 gene expression

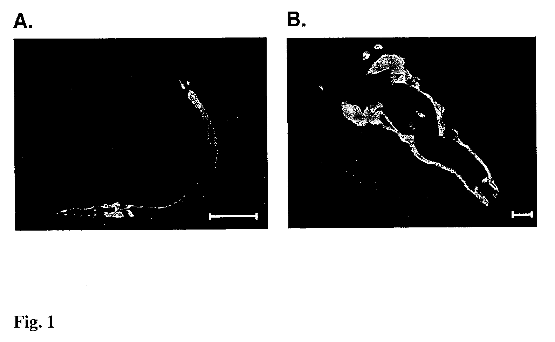

The *Ce-ncs-1* gene, located on the left arm of chromosome X, encodes *Ce*-NCS-1, a small acidic protein composed of 192 amino acids (molecular mass of 22 kDa) that binds 3 calcium ions via 4 putative EF-hands (De Castro et al., 1995). Cellular distribution of *Ce*-NCS-1 was determined by light and immunofluorescence microscopy studies using a transgenic line (XA411) expressing the green fluorescent protein (GFP) under the control of the *Ce-ncs-1* promoter region (Figure 1).

The *ncs-1:* GFP reporter gene was constructed by subcloning a *ncs-1* 3100 bp promotor comprising the sequence of the ncs-1 promoter region: 
agctttactgttttgaactaatcatcaattagctccacctactttaactagatctgttaa
caacccatgtagtgatagcttccctcattttcaaaccaatcagcagttaggtcaatctattt
ctaaaccaatgagcaactgactccgcctgttgtgaaccaatcaacaaattagctctgccttt
tttgaaaaaatcaataatttgccttgaccagcagaggaaagaaaagcgacgttaatagctga
ttaatcttgctacacggaacacggaacaaatttcaagaaagtatattctatcaataaaaaa
ctattactttgtaccgagtattgtgaaaaatcatgaatttctgtaaatgtttaatttgtaga
aacatgatctgtcgccgaaatctgcgcgaaagttgtgtggatcattatttcgttaagtggaa
acatgatctatttgctctttttgatgaaagaaacattcccaattatctgggtttcctgaa
aacttttcagtctatgttactgctgttttaatttaatcttttactggaagtcacgtttaaaa
ttggtttaaagatttattcaattttataagatttaaaaaattgtaggttgaaaattttca
gtcagagcttcgaaaagtttgggataccgtatatcctctattagtaaggcgccgttattagt
tttgcacctccattagttttgcatcaaattaggtgtccgaaaattagttttgcataccttac
taatagaggaaatacgttttcgtttgctccaatttttgtttttttttataaggacagagt
aatttctatttttttcgtattccaataattaaaatataatcagaaaataaaatcgtaaaa
aataatatgttacgtagacactcacaatcaggtaggcacaacgcatttgggtaatcttctgg
gcaaagtttgatgcatttttcccaacccagataaaagtaaaaaaaacatctaaaaagtat
caatccccaaaaaaatttgatcattttccagagctttgctctcttaaaactgcttttga
tttcttattcacgtgaaacaattgatgttgctccgatgcacaatgtgaactttgagggttt
tctgagccattagccactgacccaaaatgtgcagtctggaagatattaatttttgcttttt
ttctagaagttttcttgcagtgtttgaaagttttaagacctctcatttgccatcttactatt
agtggaatttcttcaaggaatttctcaatttcaaattcctactgactggctgttttcaaaaa
attacacatcatagttttaatgaaaaatcataggtttaatcatagttgtaatggaaaaacc
aggtatattacacaagcacccaaaaaaattccagcagtggcttggttatggcgatttccggc
aatcggtcattgaccgttttcagaaaataggtttgtcacctaaaaattctaatcaggtaata
ataatagatttcgtgataggggataattcttaatagtaaactttaaaatatttttttctctt
tcaatgatatgacagattcatcttgatttccggttttgtttaagatctgaataattccaaaa
acattcatagctttgatattggttagttgtgacttagcacccaaaaataatttactttagca
gttttaattcaaaataaaataattctgcgtaaaatttctaaatttttcaactttttatcaag
atttttgtcgagtaatgctacttcatcaaaacttcttactccatcggttgctccgactttctt
ccaatccaaaacatgtaaactcaactatcttttctctattttttagagtcctccaaaaccata
tgtctgtttgcgcgtgcgtgagatattttccccctttatgcacactcatttgtggttattc
ataaaaatgaaatatacatctagagagaaaagttagagagtcgtagagaaaatagaaattgt
attgcaccatgatttttgtcttcttttttttgccttccccttggagcaaaatcgctaatcctag
 
ctacgccagtgattgggttgctatggatctcgtgcacacttgctctcatgtacatatgtatt
ttctcacatattcggttttcccctttttttgatatctatatactgccggccgccgtgcacct
catttttctctcctcgctccgcacaccatttctgtgtgcctctgacggataaactgatgggc
atccggagcttactggtgacgtttgaggcggctcttctcccctataggaagtttggaattat
ggccttgagtgactggaaaaagaagagataactcgcataaacttcatatttccccttcatt
ttgctcatcaatttttgcccttattttaccagagatttgcagaagaactagttagttacga
tgatggaacaaaatagtcaagtcctagcgcactgaccaagactaccgttttgcactgaccaa
tttttagatctgaccaaaatttttttaagcaatagcaaaaatgttttgtttgcactgaccaa
catttttagcactttattctgcaccgaccaatattctttcagatatcaactattttcctatt
gcaccaaagcatatcaaatttgatacagctttcaaaatatataatgttatttatttgttct
taagttgccgagtatattaatacaactgctattttaaaatactttgccagtttacggttgct
tgaacacccaagaaactgaaaaaaaaattcaattccaggtaaaaatgtattccactcaagcc
tcctatcctccaaaacctaagtaaattttcgaagatttagtttcttttttttcctggagttt
agttgattgtgctccctacactttgttttctttatattcttaccacttctctacccctttat
accattgagaacccgccgaaacacatcgtttttattcaattaatgtcattttattggttctc
acacccccccaatctgctttcactatattattttttttgtctagtttccgtatttgaacgttg
ctactatttttattttcagataacaaaaaagagagaatcaagttgcaaatcaaaattatttt
attagaattgttgcgaagaaggatc
 into a GFP expression vector Tu# 63 as described in Fire et al., Gene 93 (1990), 189.
Transgenic worms were generated as previously described (Mello et al., 1991). The marker *rol-6* (plasmid pRF4) and the ncs-1::GFP construct were co-injected into gonads of hermaphrodite animals. Aligning GFP fluorescence images with differential interference Nomarski images allowed the identification of *ncs-1*::GFP Positive cells (see Table I).

**Table I:**

| NCS-1 positive cells and their functions | |
|---|---|
| Positive cells | Function |
| ***Sensory neurons:*** | |
| AWC (left, right) right) | **Amphid neurons. Chemotaxis to volatile odorants(benzaldehyde, butanone, isoamyl alcohol)** |
| ASE (L,R) | **Amphid neurons. Chemotaxis to soluble compounds (Na**^{**+**}**, Cl**^{**-**}**, cAMP, biotin, lysine), egg laying** |
| AWB (L,R) | **Amphid neurons. Volatile avoidance** |
| BAG (L,R) | **Sensory neurons** |
| PHB (L,R) | **Phasmid neurons** |
| AWA (L,R) | **Amphid neurons. Chemotaxis to volatile odorants (diacetyl, pyrazine, 2,4,5-trimethylthiazol)** |
| AFD (L,R) | **Amphid neurons. Isothermal tracking behavior. Thermotaxis** |
| ADF (L,R) ADF (L,R) | **Amphid neurons. Dauer formation; chemotaxis to soluble compounds (minor)** |
| ASG (L,R) | **Amphid neurons. Dauer formation (minor); chemotaxis to soluble compounds (minor)** |
| PHA (L,R) | **Phasmid neurons** |

| ***Inter-neurons:*** | |
|---|---|
| AVK (L,R) | |
| AIY (L,R) | **Isothermal tracking behavior. Thermotaxis** |

| ***Motor-neuron:*** | |
|---|---|
| RMG | **Irinervation of muscles in the head** |

| ***Muscle cell:*** | |
|---|---|
| pm1 | **Opening of the metastomal pharyngeal flaps** |

Confirmation of GFP staining and NCS-1 positive cells was obtained with antibodies against *Ce*-NCS-1. *Ce*-NCS-1 was predominantly expressed in sensory neurons (10 neuronal pairs: AWC, ASE, AWB, BAG, PHB, AWA, AFD, ADF, ASG, PHA). In addition 2 pairs of interneurons (AVK, AIY), 1 motor-neuron (RMG) and 1 muscle cell type (pm1) expressed *Ce*-NCS-1 (Table I). Most of the NCS-1-expressing neurons were associated with two sensory organs, the head amphids and tail phasmids (Figure 1A, 1B). A dendritic, axonal and cell body subcellular distribution was observed with *Ce*-NCS-1 specific antibodies.

### Example 2: Preparation of the ncs-1 knockout strain

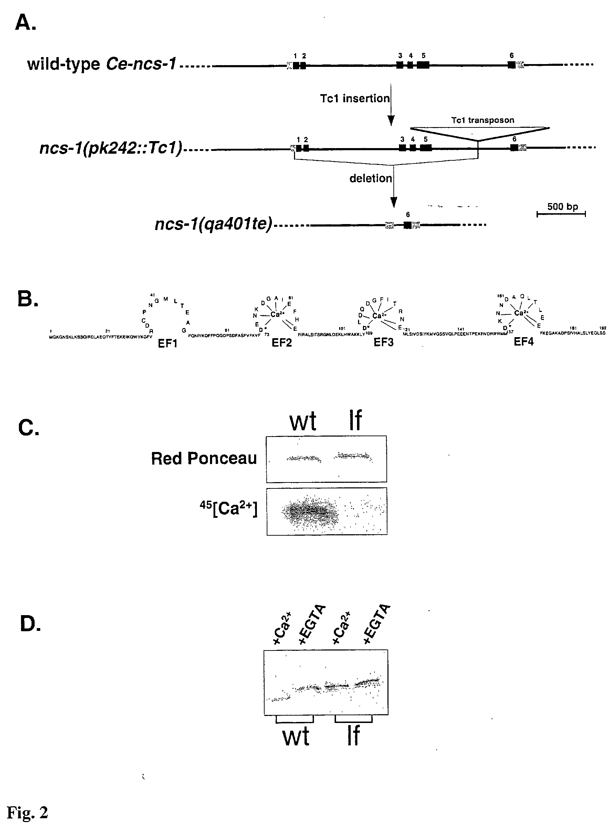

To investigate the functional role of Ce-NCS-1, knockout (KO) animals were generated. An *ncs-1* Tc1 transposon insertion mutant line (*ncs-1*(*pk242::Tc1*)) was used to isolate a deletion derivative strain *ncs-1(qa401te)* (Figure 2A).
A homozygous mutant *ncs-1(pk242)* with a Tc1 insertion located at position 5231 relative to the *ncs-1* gene fragment was obtained by PCR screening of a Tc1 insertion library, Zwaal et al., Target-selected gene inactivation in C. elegans by using a frozen transposon insertion mutant bank. Proc. Natl. Acad. Sci. USA 90 (1993), 7431-7435. Deletion derivatives were obtained as described in (Plasterk, 1995). A strain missing the genomic DNA region between exon 1 and 5 of the *ncs-1* gene was isolated (this deletion removed the first initiator ATG). This initial homozygous strain named XA401 *ncs-1(qa401te)* was back-crossed five times with N2 wild type animals (final name XA406).
The null *ncs-1* animals were viable, their developmental timing was normal although they are slightly dumpy, and the NCS-1 protein was no longer present in these KO animals (Figure 3C). Since 8/10 pairs of NCS-1 positive neurons are known to be involved in chemotaxis and volatile odorant avoidance, several classes of odor responses were measured with the KO strain. Surprisingly, null *ncs-1* mutant animals behaved like wild-type worms suggesting that calcium signaling via NCS-1 is not involved in *C. elegans* odorant detection, or that other calcium sensors in olfactory neurons can substitute or compensate for the lack of NCS-1.

### Example 3: Thermotaxis tracking behavior assay

As a cold-blooded animal, viable and fertile only within a limited temperature range (∼12-26°C), *C. elegans* has efficient thermosensory behaviors including thermal avoidance for protection against exposure to noxious temperature (Wittenburg and Baumeister, 1999), and thermotaxis for the perception of physiological (< 0.1°C) changes in local temperature (Mori, 1999). Worms learn to associate a given temperature (the growth temperature) with the presence of food during a conditioning period (acquisition) of several hours (Hedgecock and Russell, 1975). This associative conditioning is reflected by a unique phenotype, the isothermal tracking (IT) behavior, which can be observed on unseeded plates with a radial gradient of temperature with a single animal migrating to the precise growth temperature (+/-0.2°C) (Hedgecock and Russell, 1975) and then moving isothermally. When the association is disrupted (by food exhaustion), the IT behavior is conserved for several hours (extinction period) then a searching mode is activated and the worms will cross isotherms randomly to seek food at other temperatures (Mori, 1999). But a change in temperature will not lead to a random searching mode, but rather a slow reacquisition of the association between food and the new temperature. As Ce-NCS-1 was found in AFD and AIY, two neurons of the thermotaxis neural circuit, *ncs-1(qa401te)* KO worms were tested for IT behavior at 20°C (measurement as percentage of worms performing isothermal tracks at 20°C).

Briefly, 20-30 worms were grown overnight at a constant temperature of 20°C (the conditioned stimulus) in presence of a fresh lawn of the bacteria strain OP50 (the unconditioned stimulus) on a 6 cm petri dish filled with a medium (NGM) consisting of 1.7% agar, 0.25% bacto peptone, 50mM NaCI, 25mM potassium phosphate pH 6.0. Young adults were then transferred on to a fresh plate devoid of bacteria for two minutes. Individual worm were then deposited on a 9 cm Petri dish containing 9 ml of NGM. A radial gradient of temperature was created by placing a vial containing frozen acetic acid on the bottom of the plate and incubating the plate at 26°C for 90 minutes in presence of a constant humidity of 60%. Upon removal of the animal from the plate, tracks left on the agar surface were photographed.

IT recordings of single worms were visualized after 90 minutes on testing plates as shown in Figure 3A. *Ce-ncs-1* KO animals were abnormal, showing a significant difference in behavior when compared with wild-type (WT) animals (Figure 3B). 75% +/-8% of WT animals (n=94) exhibited normal IT behavior, whereas only 31% +/-9% of *ncs-1(qa401te)* mutants (n=96) performed normally. The majority of the KO animals showed irregular IT behaviors, and based on previous descriptions of thermotaxis phenotypes by Mori and Oshima (Mori and Ohshima, 1995), were classified into five categories: 31% were cryophilic, 27% athermotactic, 6% thermophilic, 5% showed intermediate behavior (mixed athermotactic and normal phenotypes), and 31% were normal. The overall IT defects of the *ncs-1* mutants (mostly athermotactic and cryophilic) were similar to the phenotypes observed with laser-killed AFD (athermotactic and cryophilic) or AIY (mostly cryophilic) animals, or with *ttx-3* (mostly cryophilic) mutants (Hobert et al., 1997), but were clearly different from AIZ (mostly thermophilic) laser-killed animals (Mori and Ohshima, 1995).

The thermal avoidance behavior of the *ncs-1* knockout strain upon exposure to a noxious temperature was also tested. Noxious temperature causes a withdrawal reflex that differs significantly from thermotaxis behavior, involves different neurons and is influenced by mutations in distinct genes (Wittenburg and Baumeister, 1999). The behavior of the *ncs-1(qa401te)* mutant did not differ from that of wild-type worms in this assay.
To ensure that the diminution of IT behavior with the KO mutant was due to the absence of *Ce*-NCS-1, a germline rescue of the KO strain was performed using either a 7kb genomic fragment transgene containing the entire *ncs-1* genomic coding region plus ∼3kb of its 5' upstream genomic sequence or a PCR fragment containing the *ncs-1* cDNA coding region plus ∼3kb of the 5' upstream genomic sequence (lines RWT, Figure 3A, B). Both transgenes were able to rescue the *ncs-1* mutant defective phenotype, resulting in restoring IT behavior in 62% +/-9% of animals (n=92, P=0.00001).

### Example 4: Calcium-binding is required for NCS-1 activity

To test whether the function of *Ce*-NCS-1 was calcium-dependent, a mutated form of NCS-1 unable to bind calcium (loss-of-function or If-NCS-1) was generated. 5µg of purified wild-type NCS-1 or If NCS-1 were subjected to electrophoresis on 10% SDS-PAGE in the presence of 5mM CaCl₂ or 2mM EGTA. Proteins were stained for visualization with Coomassie Blue (Geiser et al., 1991).
⁴⁵[Ca²⁺]-radioactive binding is readily detected with wild-type (wt) NCS-1, but not with the loss-of-function (If) *Ce*-NCS-1. A protein control with Red Ponceau staining is shown. 5µg of recombinant purified wild-type NCS-1 or loss-of-function (If) NCS-1 were run by electrophoresis on a 10% SDS-PAGE gel, blotted onto nitrocellulose membrane, and incubated with ⁴⁵[Ca²⁺] followed by several washes, and were visualized by autoradiography for 48 hours (Maruyama et al., 1984). NCS-1nt of the crucial Asp residues of the three EF-hand calcium-binding sites (positions 73, 109, and 157, Figure 2B) with Ala prevented both Ca²⁺-binding (Figure 2C) and Ca²⁺-dependent conformational shift of If-NCS-1 (Figure 2D). Lines obtained with the If-*ncs*-1 transgene (RLF) were assayed for IT behavior (Figure 3A, B), and showed a defective IT phenotype (27% +/-13%, n=78), despite the expression of the If-NCS-1 mutated protein (Figure 3C). This indicates that normal IT behavior is calcium-dependent and requires a functional, calcium-binding NCS-1 sensor.

To determine which cells require NCS-1, a mosaic rescue of the KO animals was performed using AFD (*gcy-8* (Yu et al., 1997)) or AIY (*ttx-3* (Hobert et al., 1997)) specific promotors driving the expression of *ncs-1*. A rescued IT behavior (56% +/-5%) was observed with the *ttx-3*::NCS-1 construct (RAIY animals, n=50, P=0.002), at a level similar to the rescue observed in RWT animals (Figure 3A, B). No rescue (12.5% +/-9%) in IT behavior was obtained with the *gcy-8*::NCS-1 construct (RAFD animals, n=40) (Figure 3A, B). These data strongly suggest that for normal IT behavior, NCS-1 function is required in the AIY but not AFD or any other neurons.

### Example 5: Increased level of NCS-1 affect the IT behavior of WT animals

After generating transgenic lines overexpressing NCS-1 (Tg*-ncs-1*) using the *ncs-1* cDNA under the control of the *ncs-1* promotor (presence of the construct determined by PCR), the effect in thermotaxis was measured. Figure 3B shows remarkably that NCS-1 overexpression significantly (P=0.018) increases IT thermotaxis performance (90% +/-10%, n=70) as compared to WT animal behavior (75% +/-8%). These results demonstrate that the level of NCS-1 activity can determine the efficiency of IT performance, and establish that NCS-1 is likely to be essential to the behavior and not merely permissive for IT.
To further characterize Tg*-ncs-1* worms, their IT behavior performance was studied in greater details, and compared it with WT worms. The time needed for the acquisition (learning) and the extinction period (memory) of the associative information (presence of food at the temperature of 20°C) were determined. For acquisition experiments (Figure 4A), the worms were grown for at least 12 hours in presence of food at 25°C, then were shifted individually for different time intervals onto a seeded plate at 20°C, and their IT behavior at 20°C was determined. As shown in Figure 4A, WT worms needed about 68 minutes to reach 50% of their maximal performance level, whereas Tg-*ncs-1* worms reached their 50% level after only 24 minutes. Overexpressing NCS-1 worms were therefore 2-3 times faster than the WT to learn the novel conditioning paradigm (food at 20°C). For both strains, a maximal level of performance was already reached after about 12 hours. For extinction experiments (Figure 4B), the worms were grown on seeded plates at 20°C for at least 18 hours, then individual young adult worms were washed at 20°C, transferred onto unseeded plates at 20°C for different time intervals, and their IT behavior at 20°C was determined. As shown in Fig 4B, trained WT worms needed about 3 hours to lose 50% of their maximal performance level, whereas Tg*-ncs-1* worms lost 50% of their maximal level only after about 7 hours. Therefore, the extinction period of the associative paradigm (food at 20°C) was prolonged for at least twice as long with the NCS-1 overexpressing worms as compared to WT worms. For both strains, the return to a baseline level was achieved after about 18 hours. Together, these data indicated that an elevated amount of the NCS-1 calcium sensor protein enhances not only performance, but also learning and memory functions via faster acquisition and longer retention (Fig. 5).

### References

Bartlett, S. E., Reynolds, A. J., Weible, M., Jeromin, A., Roder, J., and Hendry, I. A. (2000). Ptdlns 4-kinasebeta and neuronal calcium sensor-1 co-localize but may not directly associate in mammalian neurons. J Neurosci Res *62*, 216-24.

Braunewell, K. H., and Gundelfinger, E. D. (1999). Intracellular neuronal calcium sensor proteins: a family of EF-hand calcium-binding proteins in search of a function. Cell Tissue Res *295*, 1-12.

Cox, J. A., Durussel, I., Comte, M., Nef, S., Nef, P., Lenz, S. E., and Gundelfinger, E. D. (1994). Cation binding and conformational changes in VILIP and NCS-1, two neuron-specific calcium-binding proteins. J. Biol. Chem. *269*, 32807-32813.

De Castro, E., Nef, S., Fiumelli, H., Lenz, S. E., Kawamura, S., and Nef, P. (1995). Regulation of rhodopsin phosphorylation by a family of neuronal calcium sensors. Biochem. Biophys. Res. Commun. *216*, 133-140.

Fontana, G., and Blaustein, M. P. (1993). Calcium buffering and free Ca2+ in rat brain synaptosomes. J Neurochem *60*, 843-50.

Geiser, J. R., van Tuinen, D., Brockerhoff, S. E., Neff, M. M., and Davis, T. N. (1991). Can calmodulin function without binding calcium? Cell *65*, 949-59.

Hedgecock, E. M., and Russell, R. L. (1975). Normal and mutant thermotaxis in the nematode Caenorhabditis elegans. Proc Natl Acad Sci U S A *72*, 4061-5.

Hendricks, K. B., Wang, B. Q., Schnieders, E. A., and Thorner, J. (1999). Yeast homologue of neuronal frequenin is a regulator of phosphatidylinositol-4-OH kinase. Nat Cell Biol *1*, 234-41.

Hobert, O., Mori, I., Yamashita, Y., Honda, H., Ohshima, Y., Liu, Y., and Ruvkun, G. (1997). Regulation of interneuron function in the *C. elegans* thermoregulatory pathway by the ttx-3 LIM homeobox gene. Neuron *19*, 345-57.

lacovelli, L., Sallese, M., Mariggio, S., and de Blasi, A. (1999). Regulation of G-protein-coupled receptor kinase subtypes by calcium sensor proteins. Faseb J *13*, 1-8.

Martone, M. E., Edelmann, V. M., Ellisman, M. H., and Nef, P. (1999). Cellular and subcellular distribution of the calcium-binding protein NCS-1 in the central nervous system of the rat. Cell Tissue Res *295*, 395-407.

Maruyama, K., Mikawa, T., and Ebashi, S. (1984). Detection of calcium binding proteins by 45Ca autoradiography on nitrocellulose membrane after sodium dodecyl sulfate gel electrophoresis. J Biochem (Tokyo) *95*, 511-9.

McFerran, B. W., Graham, M. E., and Burgoyne, R. D. (1998). Neuronal Ca2+ sensor 1, the mammalian homologue of frequenin, is expressed in chromaffin and PC12 cells and regulates neurosecretion from dense-core granules. J Biol Chem *273*, 22768-72.

Mello, C. C., Kramer, J. M., Stinchcomb, D., and Ambros, V. (1991). Efficient gene transfer in C.elegans: extrachromosomal maintenance and integration of transforming sequences. Embo J *10*, 3959-70.

Milner, B., Squire, L. R., and Kandel, E. R. (1998). Cognitive neuroscience and the study of memory. Neuron *20*, 445-68.

Mori, I. (1999). Genetics of chemotaxis and thermotaxis in the nematode Caenorhabditis elegans. Annu Rev Genet *33*, 399-422.

Mori, I., and Ohshima, Y. (1995). Neural regulation of thermotaxis in Caenorhabditis elegans. Nature *376*, 344-8.

Nef, P. (1996). Neuron specific calcium sensors: The NCS subfamily. In Guidebook to the calcium-binding proteins, M. R. Celio, ed. (Oxford: Sambrook and Tooze Publication), pp. 94-98, 112-114.

Olafsson, P., Wang, T., and Lu, B. (1995). Molecular cloning and functional characterization of the Xenopus Ca(2+)- binding protein frequenin. Proc Natl Acad Sci U S A *92*, 8001-5.

Paterlini, M., Revilla, V., Grant, A. L., and Wisden, W. (2000). Expression of the neuronal calcium sensor protein family in the rat brain. Neuroscience *99*, 205-16.

Plasterk, R. H. (1995). Reverse genetics: from gene sequence to mutant worm. Methods Cell Biol *48*, 59-80.

Pongs, O., Lindemeier, J., Zhu, X. R., Theil, T., Engelkamp, D., Krah-Jentgens, I., Lambrecht, H. G., Koch, K. W., Schwemer, J., Rivosecchi, R., Mallart, A., Galceran, J., Canal, I., Barbas, J. A., and Ferrus, A. (1993). Frequenin, a novel calcium-binding protein that modulates synaptic efficacy in the drosophila nervous system. Neuron *11,* 15-28.

Putkey, J. A., Sweeney, H. L., and Campbell, S. T. (1989). Site-directed mutation of the trigger calcium-binding sites in cardiac troponin C. J Biol Chem *264*, 12370-8.

Rivosecchi, R., Pongs, O., Theil, T., and Mallart, A. (1994). Implication of frequenin in the facilitation of transmitter release in Drosophila. J Physiol (Lond) *474*, 223-32.

Schaad, N. C., de Castro, E., Nef, S., Hegi, S., Hinrichsen, R., Martone, M. E., Ellisman, M. H., Sikkink, R., Rusnak, F., Sygush, J., and Nef, P. (1996). Direct modulation of calmodulin targets by the neuronal calcium sensor NCS-1. Proc. Natl. Acad. Sci. USA *93*, 9253-9258.

Wittenburg, N., and Baumeister, R. (1999). Thermal avoidance in Caenorhabditis elegans: an approach to the study of nociception. Proc Natl Acad Sci U S A *96*, 10477-82.

Yazejian, B., Sun, X. P., and Grinnell, A. D. (2000). Tracking presynaptic Ca2+ dynamics during neurotransmitter release with Ca2+-activated K+ channels. Nat Neurosci *3*, 566-71.

Yu, S., Avery, L., Baude, E., and Garbers, D. L. (1997). Guanylyl cyclase expression in specific sensory neurons: a new family of chemosensory receptors. Proc Natl Acad Sci U S A *94*, 3384-7.

It will be clear that the invention may be practiced otherwise than as particularly described in the foregoing description and examples. Numerous modifications and variations of the present invention are possible in light of the above teachings and, therefore, are within the scope of the appended claims.

The entire disclosure of each document cited (including patents, patent applications, journal articles, abstracts, laboratory manuals, books, or other disclosures) in the Background of the Invention, Description, and Examples is hereby incorporated herein by reference.

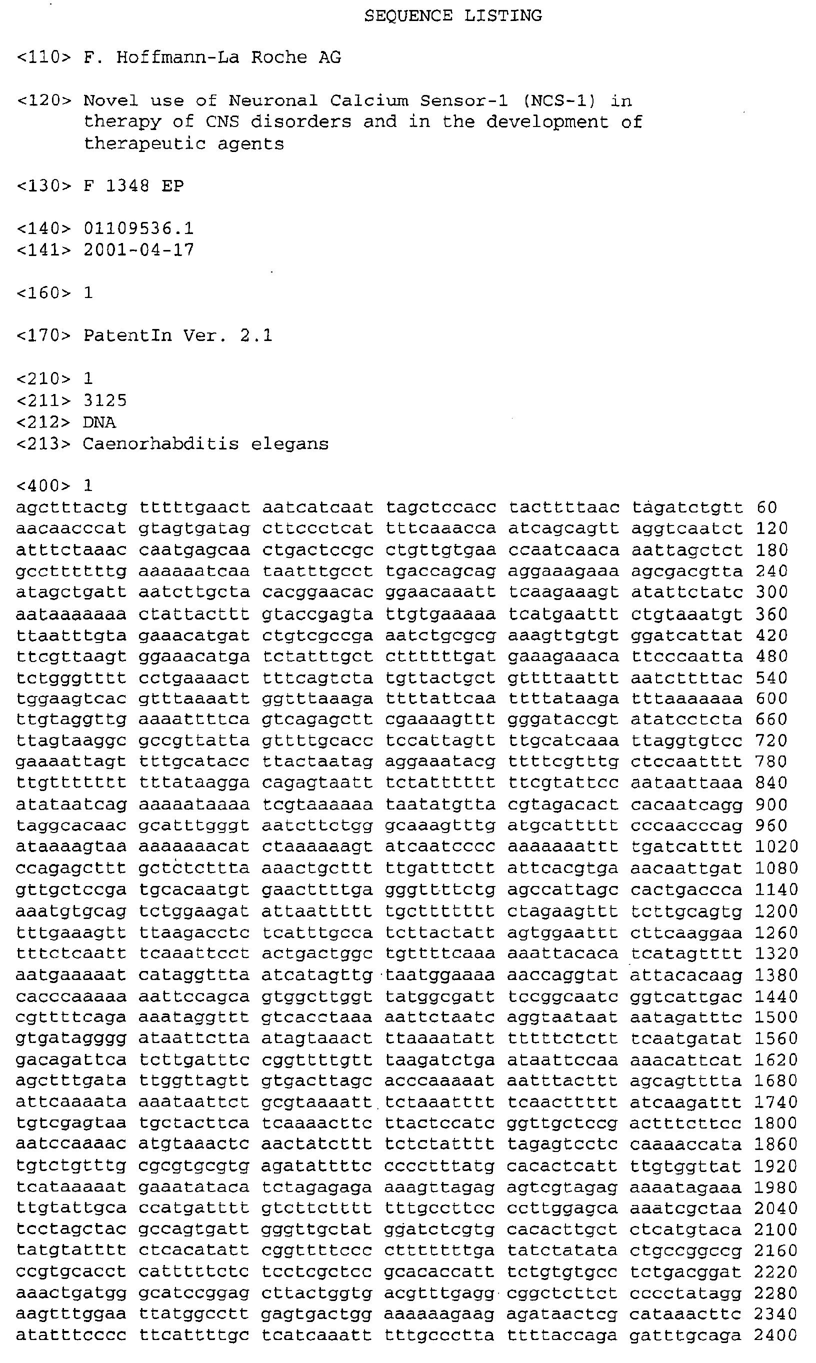

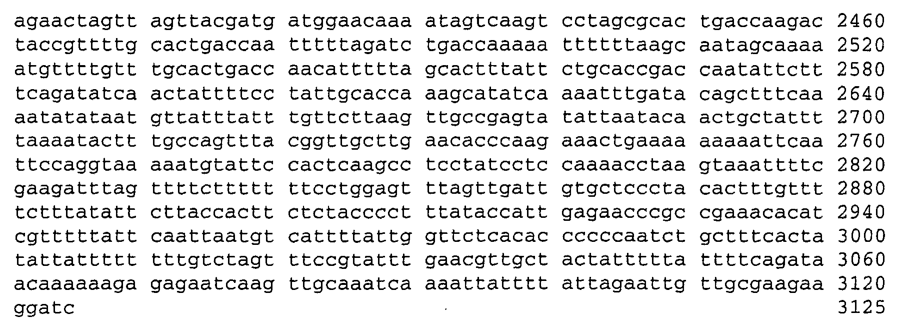

## Claims

1. A pharmaceutical composition comprising an agonist/activator or antagonist/inhibitor of neuron-specific calcium sensor-1 (NCS-1), and a pharmaceutically acceptable carrier.

2. Use of an agonist/activator or of an antagonist/inhibitor of neuron-specific calcium sensor-1 (NCS-1) or a pharmaceutically acceptable salt thereof for the preparation of a composition for the treatment of a CNS disorder or for improving cognition of a subject, or for modulating neurotransmitter release.

3. The use of claim 2, wherein said CNS disorder is Schizophrenia, Alzheimer's Disease, Parkinson's Disease, major depression, bipolar disorder, anxiety disorders, appetite disorders, sleep disorders, insomnia, attention deficit hyperactivity disorder or hyperactivity.

4. The pharmaceutical composition of claim 1 or the use of claim 2 or 3, wherein the agonist/activator is or is derived from an NCS-1 polypeptide, an anti-NCS-1 antibody, a transcription regulator of the *ncs*-1 gene, a ligand binding molecule, a calcium mimetic or a derivative of a calmodulin activator.

5. The pharmaceutical composition of claim 1 or the use of claim 2 or 3, wherein the antagonist/inhibitor is or is derived from an NCS-1 polypeptide, an anti-NCS-1 antibody, an *ncs*-1 antisense nucleic acid molecule, a ligand binding molecule, a calcium chelator or a calmodulin inhibitor.

6. The pharmaceutical composition of claim 1, 4 or 5 or the use of any one of claims 2 to 5, wherein said agonist/activator facilitates and said antagonist/inhibitor interferes with calcium binding of NCS-1 or changes the conformation/function of NCS-1.

7. A method of identifying and obtaining a drug candidate for therapy of a behavioral disorder or for improving learning and/or memory of a subject, said method comprising the steps of
(a) screening a cell, tissue or non-human animal with a compound to be screened under conditions to permit neuron-specific calcium sensor-1 (NCS-1) activity; and
(d) determining NCS-1 activity of said treated cell, tissue or non-human animal, wherein a difference in NCS-1 activity compared with a corresponding control cell, tissue or animal is indicative for a drug candidate; and optionally
(e) obtaining the drug candidate determined to alter NCS-1 activity in step (b).

8. The method of claim 7, wherein said NCS-1 activity is calcium binding or a change of conformation/function.

9. The method of claim 7 or 8, wherein said cell, tissue or non-human animal is a transgenic cell, tissue or non-human animal which displays a substantially reduced or enhanced level of neuron-specific calcium sensor-1 (NCS-1) activity compared to a corresponding wild-type animal.

10. The method of claim 9, wherein for the transgenic non-human animal said difference in NCS-1 activity results in a difference in behavior.

11. The method of claim 9 or 10, wherein said transgenic non-human animal displaying a reduced level of neuron-specific calcium sensor-1 (NCS-1) activity comprises at least one mutant allele of the NCS-1 encoding gene or a corresponding trans-dominant allele of a different gene.

12. The method of claim 11, wherein said transgenic non-human animal is a *ncs*-1 knock-out animal.

13. The method of claim 11 or 12, wherein said transgenic non-human animal is *C. elegans* and said behavior is isothermal tracking (IT).

14. The method of any one of claims 7 to 13, wherein said compound to be screened is derived from an NCS-1 polypeptide, an NCS-1 antisense molecule, an anti-NCS-1 antibody, a transcription regulator of the *ncs*-1 gene, a ligand binding molecule, a calcium mimetic or a derivative of a calmodulin activator or inhibitor.

15. The method of any one of claims 7 to 14, wherein said drug candidate is a lead compound.

16. A method of producing a drug comprising the steps of the method of any one of claims 7 to 15; and
(i) synthesizing the drug candidate identified in step (b) or obtained in step (c) or an analog or derivative thereof in an amount sufficient to provide said drug in a therapeutically effective amount to a subject; and/or
(ii) combining the drug candidate identified in step (b) or obtained in step (c) or an analog or derivative thereof with a pharmaceutically acceptable carrier

17. A pharmaceutical composition comprising a drug or drug candidate identified or obtained in the method of any one of claims 7 to 16 or a racemate, enantiomer, diastereomer, tautomer, mixture of diastereomers or pharmaceutically acceptable salt of any one those, wherein said drug or drug candidate is a modulator of NCS-1 activity.

18. The pharmaceutical composition of claim 17, wherein said drug facilitates or interferes with calcium binding of NCS-1 or changes conformation of NCS-1.

19. A transgenic non-human animal as defined in any one of claims 11 to 13 or a transgenic cell or tissue thereof.

20. Use of a neuron-specific calcium sensor-1 (NCS-1) or a biologically active fragment thereof, a nucleic acid molecule encoding NCS-1 or nucleic acid molecule of at least 15 nucleotides in length hybridizing to a *ncs*-1 gene, an anti-NCS-1 antibody, a cell of claim 9, or of an NCS-1 activity assay for a method of obtaining, identifying and/or profiling a drug candidate for therapy of a CNS disorder or for modulating cognition of subject.

21. Use of a cell, a tissue, a non-human animal and/or a drug candidate for method of any one of claims 7 to 16.

22. A method of diagnosing a pathological condition or a susceptibility to a pathological condition in a subject related to a CNS disorder:
(a) determining the presence or absence of a mutation in the polynucleotide encoding neuron-specific calcium sensor-1 (NCS-1); and
(b) diagnosing a pathological condition or a susceptibility to a pathological condition based on the presence or absence of said mutation.

23. A method of diagnosing a pathological condition or a susceptibility to a pathological condition in a subject related to a CNS disorder comprising:
(a) determining the presence or amount of expression of a neuron-specific calcium sensor-1 (NCS-1) polypeptide in a biological sample; and
(b) diagnosing a pathological condition or a susceptibility to a pathological condition based on the presence or amount of expression of the polypeptide.

24. A method of treating a patient in need of such treatment for a disorder which is mediated by neuron-specific calcium sensor-1 (NCS-1), comprising administration of a drug or drug candidate identified or obtained in the method of any one of claims 7 to 16.

25. A method for treating a CNS disorder in a subject or improving cognition of a subject, which method comprises administering to the subject an effective amount of a therapeutic agent to increase the level and/or activity of NCS-1, so as to improve or restore calcium signaling in the subject.

26. The use of any one of claims 2 to 4 or 20, or the method of any one of claims 7 to 16 or 22 to 25, wherein said disorder is or is related to Alzheimer's disease, Parkinson's disease, age-associated cognition deficits, or memory loss or a learning deficiency.

27. A kit useful for the method of any one of claims 7 to 16, said kit comprising a neuron-specific calcium sensor-1 (NCS-1) or a biologically active fragment thereof, a nucleic acid molecule encoding NCS-1 or nucleic acid molecule of at least 15 nucleotides in length hybridizing to a *ncs*-1 gene, an anti-NCS-1 antibody, a cell of claim 9, or a cell, a tissue or a non-human animal.

28. A kit useful for the method of claim 22 or 23, said kit comprising a neuron-specific calcium sensor-1 (NCS-1) or a biologically active fragment thereof, a nucleic acid molecule encoding NCS-1 or nucleic acid molecule of at least 15 nucleotides in length hybridizing to a *ncs*-1 gene, an anti-NCS-1 antibody or a cell of claim 9.

29. The kit of claim 27 comprising *C. elegans* displaying a reduced or enhanced level of NCS-1 activity, and optionally a corresponding control animal.
